# EUROPEAN PATENT APPLICATION

(11) **EP 4 497 829 A1**
(43) Date of publication of application: **29.01.2025**
(21) Application number: 23382795.5
(22) Date of filing: 28.07.2023
(51) Int. Cl.: C12N 15/82

(54) **MINIMAL PRECURSORS OF SYN-TASIRNAS**

(71) Applicant: CONSEJO SUPERIOR DE INVESTIGACIONES CIENTIFICAS (CSIC), 28006 Madrid (ES); Universitat Politècnica de València, 46022 Valencia (ES)
(72) Inventor: CARBONELL OLIVARES, Alberto Tomás, 46022 VALÈNCIA (ES); DARÓS ARNAU, José Antonio, 46022 VALÈNCIA (ES); GONZÁLEZ CISNEROS, Adriana Estela, 46022 VALÈNCIA (ES)
(74) Representative: ZBM Patents - Zea, Barlocci & Markvardsen

(57) **Abstract**

The invention provides a viral vector comprising a heterologous nucleotide sequence that encodes or corresponds to an RNA sequence S1 that has a length of 52-350 ribonucleotides and that comprises regions R1, R2 and R3 directly linked to each other as indicated in formula (i):

(i) 5'-R1-R2-R3-3',

wherein:
- region R1 consists of the target site of a sRNA,
- region R2 consists of an RNA sequence of 11 ribonucleotides, and
- Region R3 consists of the RNA sequence of one or more siRNAs, wherein when R3 is formed by more than one siRNA sequence, the siRNA sequences are directly linked to each other. The invention also provides a virus encoded by or comprising the viral vector, a plasmid comprising or encoding the viral vector, a bacterium comprising the plasmid, a plant comprising the viral vector, the virus or the bacterium, a plant extract from the plant, and a method for obtaining the plant.

## Description

### Technical Field

The present invention relates to the field of biotechnology. More particularly it relates to syn-tasiRNA minimal precursors that can be expressed in plants through viral vectors and to non-transgenic plants expressing them.

### Background Art

Small RNAs (sRNAs) are a class of short, non-coding regulatory RNAs that have emerged as critical components of defense regulatory networks across plant kingdoms. Many sRNA-based technologies, such as host-induced gene silencing (HIGS), spray-induced gene silencing (SIGS), artificial microRNA (amiRNA) have been developed as disease control strategies in plants and for improving important traits of crops.

In particular, to breed non-genetically modified (GMO), disease tolerant crops, SIGS has appeared a promising technology to enhance crop resistance to diseases or for modifying traits of interest in crops. However, one of the main limitations of the application of SIGS is the rapid degradation of naked RNAs.

Artificial miRNAs (amiRNAs) are miRNAs designed for redirecting the endogenous miRNA biogenesis and silencing machineries of plants so that the expression of specific target RNAs is suppressed with high efficacy and specificity (with no predicted off-targets). However, a current limitation of the amiRNA technology is the relatively long length of the precursors that allow the production of the amiRNAs *in vivo.* Additionally, in order to target more than one mRNA sequence in a plant, several amiRNA precursors have to be transfected, each one addressed to one mRNA target sequence, which complicates even more the applicability of the technique to methods that require silencing several mRNAs at the same time.

Therefore, there is need in the field of new strategies for the efficient silencing of specific genes, especially when more than one sequence has to be silenced, and especially, in a transgene-free manner.

### Summary of Invention

The present inventors have developed minimal syn-tasiRNA precursors that comprise the sequences that are sufficient and necessary for the expression of the correct syn-tasiRNAs they comprise in virtually any plant.

As shown in the examples below, the minimal syn-tasiRNA precursors developed by the inventors provide various advantages over known syn-tasiRNA precursors such as those derived from the full-length *AtTAS1C* precursor. For example, efficient silencing of a gene of interest in virtually any plant can be achieved with the minimal precursor, without the need of co-expressing a particular miRNA in said plant, as for instance miRNA173 in the case of known precursors based on the full-length *AtTAS1C* precursor. In addition, as opposed to, for instance, a precursor based on the full-length *AtTAS1c* sequence, the minimal precursors developed by the inventors produce functional syn-tasiRNAs to silence genes when expressed from a viral vector. Non-transgenic plant tissues can thus be obtained wherein one or several genes of interest are silenced.

Moreover, viral vector mediated gene silencing with the minimal precursors can be achieved not only against endogenous genes in virtually any plant, but also against genes in the genome of a plant pathogen, such as the TSWV. Therefore, the minimal precursors are also useful to confer resistance to plant pathogens in virtually any plant.

Thus, in a first aspect, the invention provides a viral vector comprising a heterologous nucleotide sequence that encodes or corresponds to an RNA sequence S1 that has a length of 52-350 ribonucleotides and that comprises regions R1, R2 and R3 directly linked to each other as indicated in formula (i):

(i) 5'-R1-R2-R3-3',

wherein:
- Region R1 consists of the target site of a sRNA,
- Region R2 consists of an RNA sequence of 11 ribonucleotides, and
- Region R3 consists of the RNA sequence of one or more siRNAs, wherein when R3 is formed by more than one siRNA sequence, the siRNA sequences are directly linked to each other.

In a second aspect, the invention provides a viral vector comprising a sequence that is the reverse complement of the sequence of the viral vector as defined in the first aspect of the invention.

In a third aspect, the invention provides a virus encoded by or comprising the viral vector as defined in the first or the second aspect of the invention.

In a fourth aspect, the invention provides an RNA polynucleotide comprising the RNA sequence S1 as defined in the first aspect of the invention, provided that the RNA polynucleotide does not comprise:
(i) in the region upstream of region R1, the sequence immediately upstream the miR173 target site in the *Arabidopsis thaliana trans-acting siRNA1C* (*AtTAS1C*) primary precursor, and/or
(ii) in the region downstream of region R3, the sequence immediately downstream sequence SEQ ID NO. 26 in the *AtTAS1C* primary precursor.

In a fifth aspect, the invention provides a DNA polynucleotide encoding the polynucleotide as defined in the fourth aspect of the invention.

In a sixth aspect, the invention provides a plasmid comprising a sequence that encodes or corresponds to the sequence of the viral vector as defined in the first or second aspect of the invention, or the sequence encoding the polynucleotide as defined in the fourth aspect of the invention.

In a seventh aspect, the invention provides a bacterium that comprises the plasmid as defined in the sixth aspect of the invention.

In a eighth aspect, the invention provides a plant that comprises the viral vector as defined in the first or second aspect of the invention, the virus as defined in the third aspect of the invention, the polynucleotide as defined in the fourth or fifth aspect, the plasmid as defined in the sixth aspect and/or the bacterium as defined in the seventh aspect.

In ninth aspect, the invention provides a plant extract from the plant as defined in the eighth aspect.

In a tenth aspect, the invention provides a method for obtaining the plant as defined in the eighth aspect, the method comprising the step of:
(i) transfecting a plant with the plasmid as defined in the sixth aspect, particularly by agroinfiltration with the bacterium as defined in the seventh aspect, or alternatively,
(ii) contacting the surface of at least one organ of a plant with the virus as defined in the third aspect.

In an eleventh aspect, the invention provides a method for obtaining the plant extract according to the ninth aspect that comprises the steps of:
(i) Grinding to powder at least one organ or a section thereof of the plant as defined in the eighth aspect, and
(ii) Homogenizing the powder obtained in step (i) in a buffer.

In a twelfth aspect, the invention provides a kit of parts comprising the viral vector as defined in the first or second aspect, the virus as defined in the third aspect, the polynucleotide as defined the fourth or fifth aspect, and/or the plasmid as defined in the sixth aspect.

In a thirteenth aspect, the invention provides a device comprising the virus as defined in the third aspect, the bacterium as defined in the seventh aspect, or the plant extract as defined in the ninth aspect.

In a fourteenth aspect, the invention provides a method for obtaining the viral vector as defined in the first or second aspect that comprises:
i) Transfecting an organ of a plant with the plasmid as defined in sixth aspect, particularly by agroinfiltration with the bacterium as defined in the seventh aspect,
ii) Allowing the plant to grow for at least 1 week, and
iii) Isolating the viral vector comprised in the organ transfected in step (i), from the plant obtained from step (ii).

In a fifteenth aspect, the invention provides a method for obtaining the virus as defined in the third aspect that comprises:
i) Transfecting an organ of a plant with the plasmid as defined in sixth aspect, particularly by agroinfiltration with the bacterium as defined in the seventh aspect,
ii) Allowing the plant to grow for at least 1 week, and
iii) Isolating the viral vector comprised in the organ transfected in step (i), from the plant obtained from step (ii).

In a sixteenth aspect, the invention provides a method for protecting a plant from one or more plant pathogens, the method comprising:
(i) transfecting a plant with the plasmid as defined in the sixth aspect, particularly by agroinfiltration with the bacterium as defined in the seventh aspect, or alternatively,
(ii) contacting the surface of at least one organ of a plant with the virus as defined in the third aspect.

In a seventeenth aspect, the invention provides a method for the treatment of one or more plant pathogens diseases in a plant, the method comprising:
(i) transfecting a plant with the plasmid as defined in the sixth aspect, particularly by agroinfiltration with the bacterium as defined in the seventh aspect, or alternatively,
(ii) contacting the surface of at least one organ of a plant with the virus as defined in the third aspect.

In an eighteenth aspect, the invention provides a method for improving a trait of a plant, the method comprising:
(i) transfecting a plant with the plasmid as defined in the sixth aspect, particularly by agroinfiltration with the bacterium as defined in the seventh aspect, or alternatively,
(ii) contacting the surface of at least one organ of a plant with the virus as defined in the third aspect.

### Brief description of the drawings

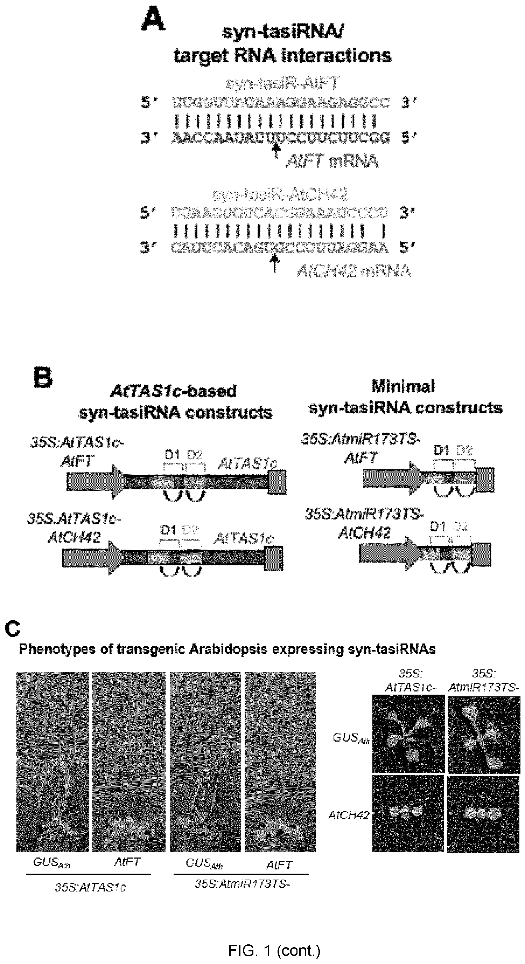

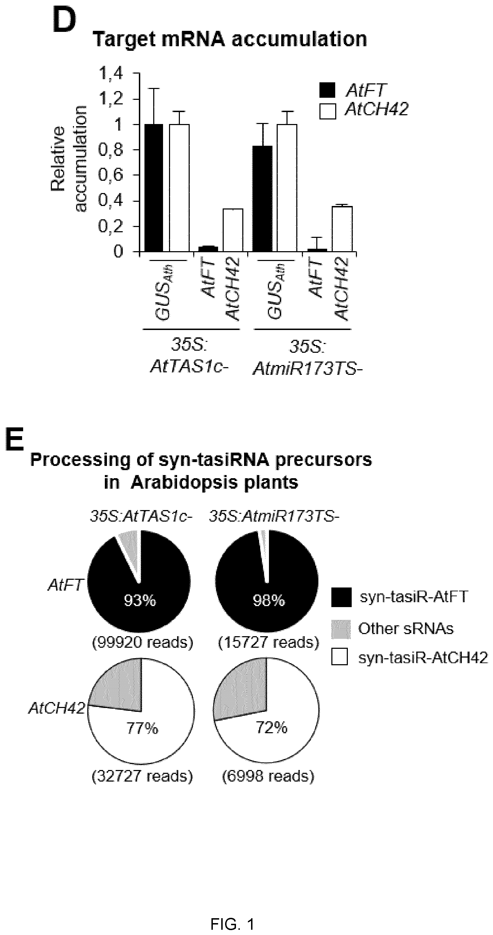

Figure 1. Silencing by syn-tasiRNAs derived from minimal precursors in *Arabidopsis thaliana.* A, Base-pairing interactions between syn-tasiRNAs and their target mRNAs. Black linear arrows indicate sRNA-guided cleavage sites. B, Diagrams of constructs expressing from D2 syn-tasiR-AtFT or syn-tasiR-AtCH42. Target site (TS) from Arabidopsis miR173 is depicted as the first light grey box after the big grey arrow from left to right, and just previous to the light grey box representing site D2. Curved black arrows indicate DCL4 processing sites. C, Phenotypes of T1 transgenic plants expressing different syn-tasiRNA constructs. D, Accumulation of target mRNA in Arabidopsis transgenic plants analyzed by RT-qPCR. E, Processing accuracy of syn-tasiRNA precursors.

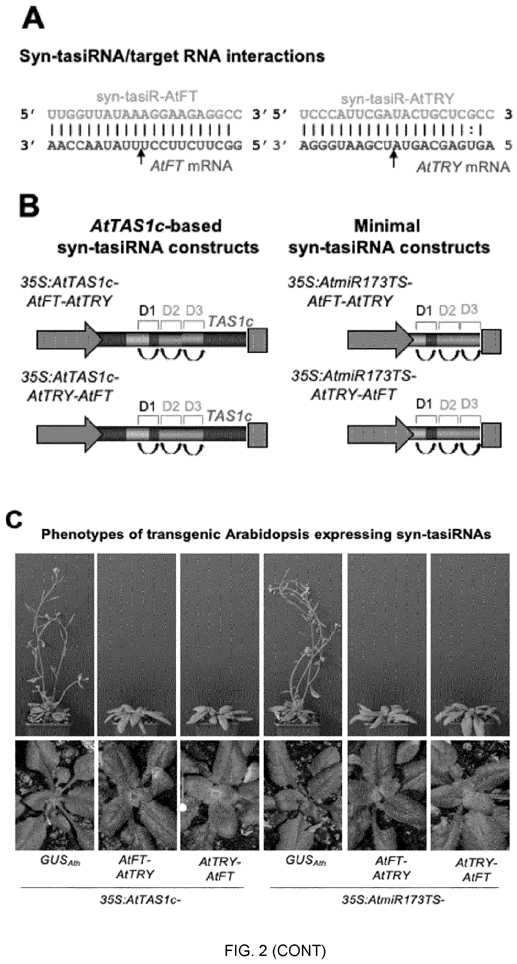

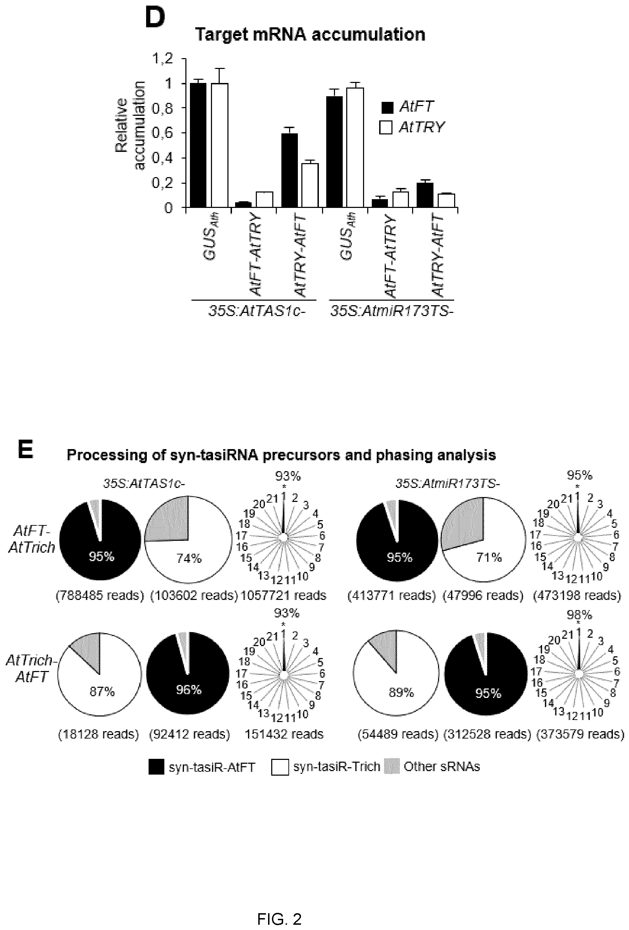

Figure 2. Silencing by syn-tasiRNAs derived from minimal precursors in Arabidopsis thaliana. A, Base-pairing interactions between syn-tasiRNAs and their target mRNAs. Black linear arrows indicate sRNA-guided cleavage sites. B, Diagrams of constructs expressing syn-tasiR-AtFT (from D2 in constructs 35S: AtTAS1c-AtFT-AtTRY and in the 35S:AtmiR173TS-AtFT.AtTRy, or from D3 in constructs 35S:AtTAS1c-AtTRY-AtFT and 35S:AtmiR173TD-AtTRY-AtFT): or syn-tasiR-AtTRY (from D3 in constructs 35S: AtTAS1c-AtFT-AtTRY and in the 35S:AtmiR173TS-AtFT.AtTRy, or from D2 in constructs 35S:AtTAS1c-AtTRY-AtFT and 35S:AtmiR173TD-AtTRY-AtFT):. Target site (TS) from Arabidopsis miR173 is depicted as the first light grey box after the big grey arrow from left to right, and just previous to the light grey box representing site D2 . Curved black arrows indicate DCL4 processing sites. Images are from agroinfiltrated leaves. C, Phenotypes of T1 transgenic plants expressing different syn-tasiRNA constructs. D, Accumulation of target mRNAs in Arabidopsis transgenic plants analyzed by RT-qPCR. E, Processing accuracy of syn-tasiRNA precursors as well as phasing analysis. Pie charts show the percentage of expected syn-tasiRNA sequences result of a correct precursor processing. Radar plots show percentages of 21-nt reads corresponding to each of the 21 registers from AtTAs1c or from minimal transcripts, with position 1 designated as immediately after the miR173-guided cleavage site.

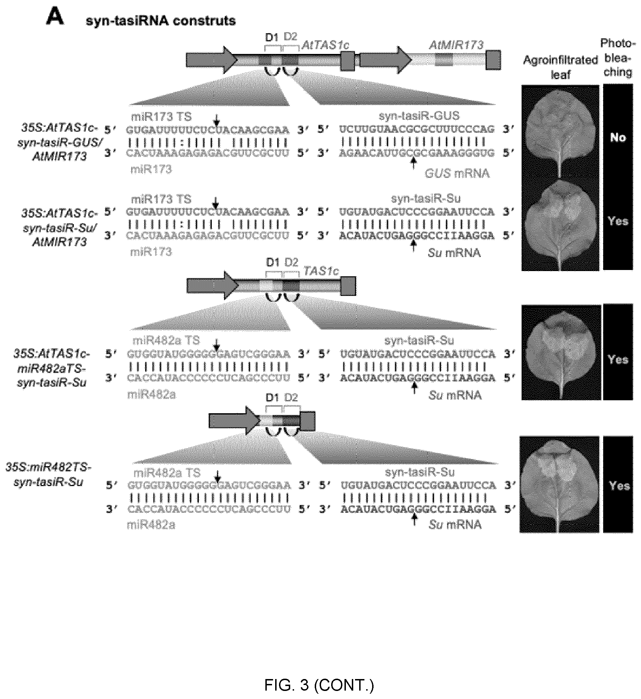

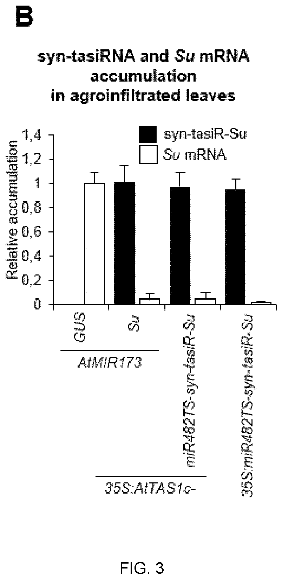

Figure 3. Silencing by syn-tasiRNAs derived from minimal precursors in Nicotiana benthamiana. A, Diagrams of constructs expressing syn-tasiR-Su or syn-tasiR-GUS. Target sites (TS) from Arabidopsis miR173 or N. benthamiana miR482 are depicted as the first light grey box after the big grey arrow from left to right, and just previous to the light grey box representing site D2. Curved black arrows indicate DCL4 processing sites. Black linear arrows indicate sRNA-guided cleavage sites. Images are from agroinfiltrated leaves. B, Accumulation of syn-tasiR-Su and Su mRNA in agroinfiltrated patches.

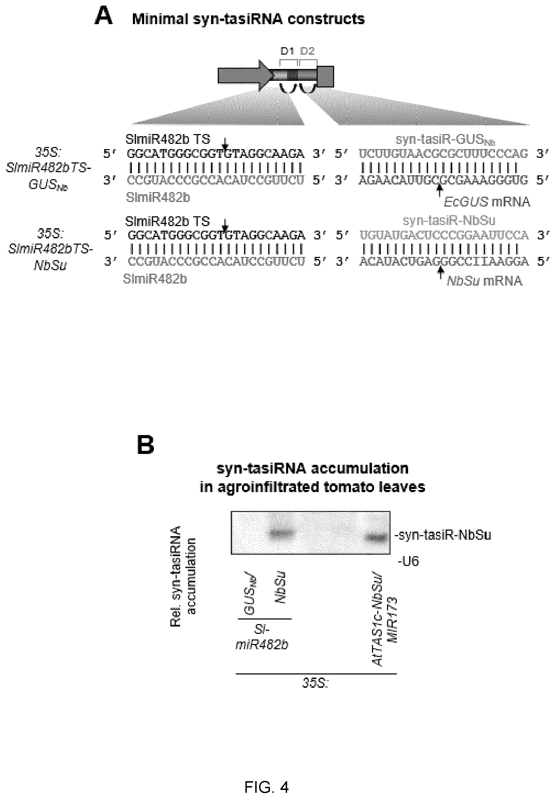

Figure 4. Silencing by syn-tasiRNAs derived from minimal precursors in Solanum lycopersicum. A, Diagrams of SlmiR482bTS-based constructs expressing syn-tasiR-NbSu or syn-tasiR-GUS. Target site (TS) from S. lycopersicum miR482b is highlighted in black, with artificial nucleotide substitution for complete canonical base-pairing with slmiR482b in nucleotides in position 3, 9, and 18 fron the 5'- to the 3'-end . Syn-tasiR-NbSu and syn-tasiR-GUS sequences are also highlighted. Curved black arrows indicate DCL4 processing sites. Black linear arrows indicate sRNA-guided cleavage sites. B, Accumulation of syn-tasiR-NbSu in agroinfiltrated patches when expressed from minimal SlmiR482b-based precursors or from full-length AtTAS1c/AtmiR173aTS-based precursors.

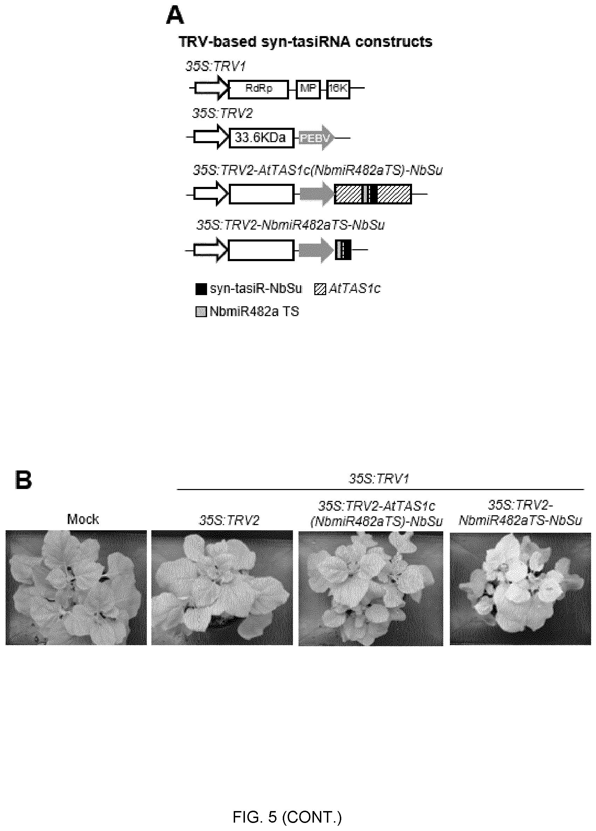

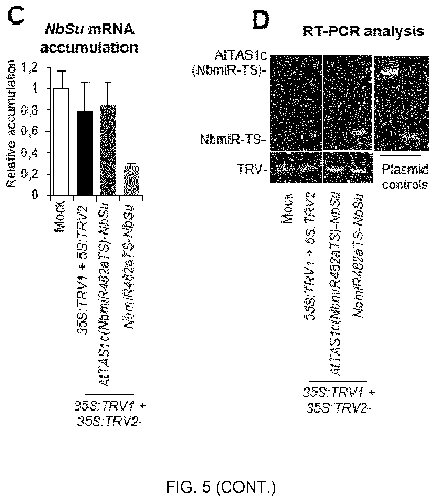

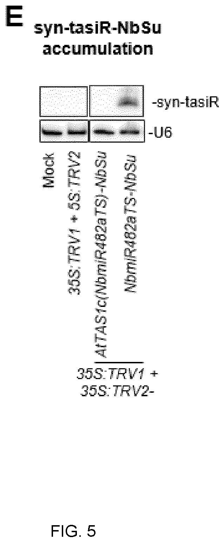

Figure 5. Silencing by syn-tasiRNAs derived from minimal precursors included in TRV in Nicotiana benthamiana. A, Diagrams of TRV2-based constructs to express syn-tasiRNAs from precursors including NbmiR482a target site. B, Photographs of agroinoculated plants at 14 dpa. C, Accumulation at 7 dpa of NbSu mRNA in apical leaves of agroinoculated plants. D, RT-PCR detection of syn-tasiRNA precursors at 14 dpa in apical leaves from agroinoculated plants. E, Northern blot analysis of syn-tasiR-NbSu accumulation in apical leaves at 7 dpa.

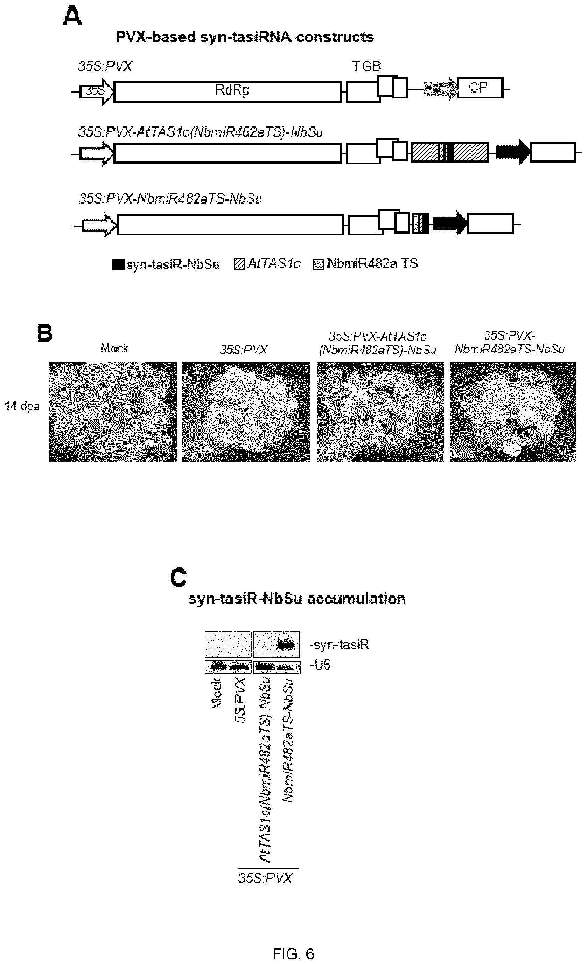

Figure 6. Silencing by syn-tasiRNAs derived from minimal precursors included in PVX in Nicotiana benthamiana. A, Diagrams of PVX-based constructs to express syn-tasiRNAs from precursors including NbmiR482a target site. B, Photographs of agroinoculated plants at 14 dpa. C, Accumulation at 7 dpa of NbSu mRNA in apical leaves of agroinoculated plants. D, RT-PCR detection of syn-tasiRNA precursors at 14 dpa in apical leaves from agroinoculated plants. E, Northern blot analysis of syn-tasiR-NbSu accumulation in apical leaves at 7 dpa.

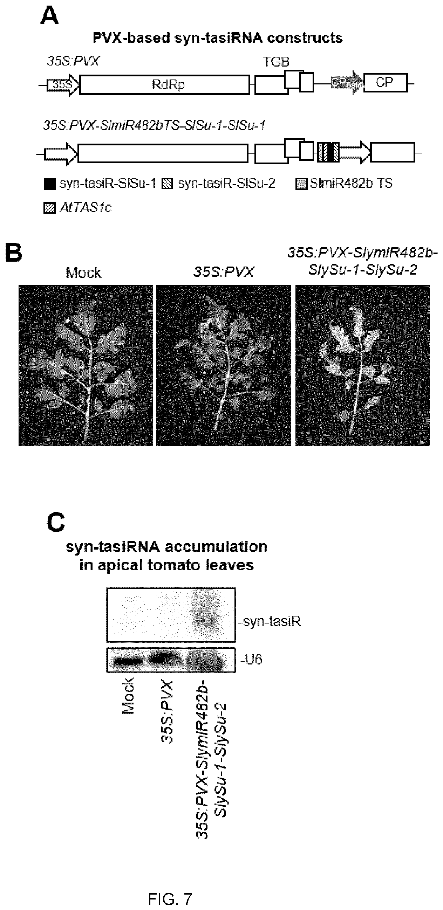

Figure 7. Silencing by syn-tasiRNAs derived from minimal precursors included in PVX in Solanum lycopersicum. A, Diagrams of PVX-based constructs to express syn-tasiRNAs from precursors including SimiR482b target site. B,Photographs of apical non agroinoculated leaves at 14 dpa. C, Accumulation at 7 dpa of SISu syn-tasiRNAs in apical leaves of agroinoculated plants.

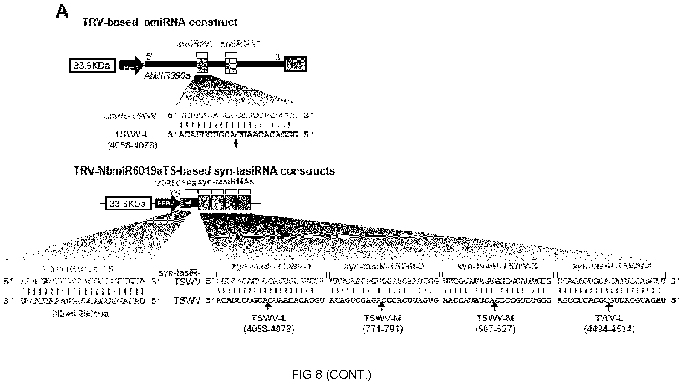

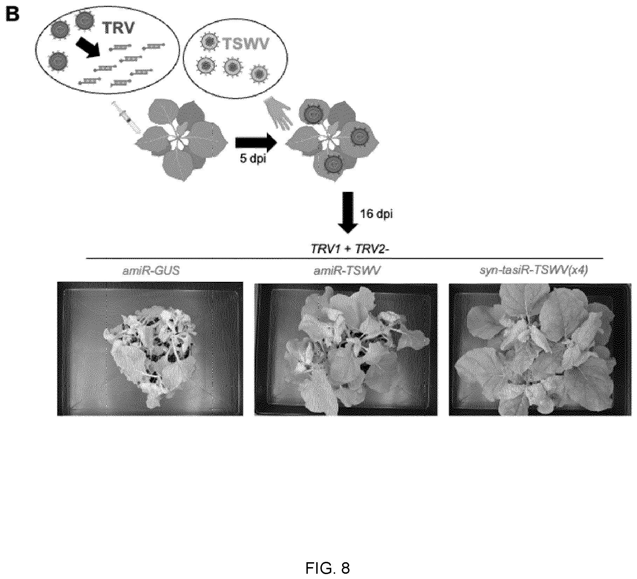

Figure 8. Vaccination of plants with TRV-based vectors expressing antiviral syn-tasiRNAs against a pathogenic virus. A, Diagrams of TRV-based constructs expressing one anti-TSWV amiRNA (amiR-TSWV) or 4 anti-TSWV syn-tasiRNAs (syn-tasiR-TSWV-1 to syn-tasiR-TSWV-4). Target site (TS) from N. benthamiana miR6019a is indicated with artificial nucleotide substitution for complete canonical base-pairing with slmiR482b in nucleotide positions 5, 18 and 20 in a 5' to 3' end direction . Syn-tasiRNAs sequences are also highlighted. Curved black arrows indicate DCL4 processing sites. Black linear arrows indicate sRNA-guided cleavage sites. B, Scheme of the experiment. Plants are agroinoculated with TRV-based constructs, and 5 days later the agroinoculated leaves are mechanically inoculated with TSWV crude extracts. Plants are monitored for symptom appearance for 2-3 weeks. Plant photographs are taken 16 days after TSWV inoculation.

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

As used herein, the indefinite articles "a" and "an" are synonymous with "at least one" or "one or more." Unless indicated otherwise, definite articles used herein, such as "the" also include the plural of the noun.

For purposes of the present invention, any ranges given include both the lower and the upper end-points of the range. Ranges given, such as concentrations and the like, should be considered approximate, unless specifically stated. The term "about" refers to a deviation of plus/minus 10 %, preferably plus/minus 5 %.

### I- Viral vectors of the invention

As described above, in a first aspect the invention provides a viral vector comprising a heterologous nucleotide sequence that encodes or corresponds to an RNA sequence S1 that has a length of 52-350 ribonucleotides and that comprises regions R1, R2 and R3 directly linked to each other as indicated in formula (i):

(i) 5'-R1-R2-R3-3',

wherein:
- Region R1 consists of the target site of a sRNA,
- Region R2 consists of an RNA sequence of 11 ribonucleotides, and
- Region R3 consists of the RNA sequence of one or more siRNAs, wherein R3 is formed by more than one siRNA sequence, the siRNA sequences are directly linked to each other.

As well understood by a skilled person, the terms "RNA sequence S1", "sequence S1", or "S1" as used herein, are equivalent and all refer to the RNA sequence S1 as described in the first aspect of the invention. The terms "RNA region R1", "region R1" or "R1" as used herein, are equivalent and all refer to the RNA region R1 as described in the first aspect of the invention. The terms "RNA region R2", "region R2" or "R2" as used herein, are equivalent and all refer to the RNA region R2 as described in the first aspect of the invention. The terms "RNA region R3", "region R3" or "R3" as used herein, are equivalent and all refer to the RNA region R3 as described in the first aspect of the invention. Additionally, the term "sRNA of the invention" as used herein, refers to the sRNA whose sequence corresponds to that of region R1 as defined in the first aspect of the invention.

The term "viral vector", or "plant viral vector", as used herein, refers to the term commonly known by an expert in the field. Viral vectors correspond to the genetic material of a virus, or to a modified genetic material of a specific virus, in which a nucleic acid sequence of interest can be inserted to express the corresponding nucleic acid sequence. Indeed, viral vectors have been commonly used to express genetic material of interest in a target cell, by inserting said genetic material of interest in the genetic material of a known virus. Modifications of the viral genetic material in the viral vectors are commonly addressed to allow or improve the expression of the nucleic acid sequence or protein sequence of interest in the target cell. Several vector design strategies, such as gene replacement, gene insertion using a duplicated subgenomic RNA (sgRNA) promoter (SGP), heterologous SGP, complementation, gene fusions, internal ribosomal entry site (IRES), and deconstructed viruses, can be used for expression of foreign nucleic acid sequences or proteins from virus vectors. Viral vectors are commonly referred to by reference to the virus whose genetic material corresponds or has been used as a template for the obtention of the viral vector. In other words, a viral vector is commonly referred to by reference to the virus from which it derives. For instance, a PVX viral vector corresponds to a viral vector that derives from the PVX virus, hence, with a sequence that corresponds to the PVX genetic material in which a nucleic acid sequence of interest can be inserted, or to the PVX virus genetic material including specific genetic modifications and in which a nucleic acid sequence of interest can be inserted. Nonlimiting examples of genetic modifications of the viral genetic material in a viral vectors include the deletion of a sequence or part of a sequence encoding a viral protein, for instance, the viral coat protein (CP), the inclusion of a heterologous promoter, such as the Pea early browning virus (PEBV) promoter or the Bamboo mosaic virus (BaMV) promoter, or the inclusion of a viral protein into the control of a heterologous promoter as just mentioned. In a particular embodiment, the viral vector of the invention comprises a heterologous promoter, particularly the Pea early browning virus (PEBV) promoter or the Bamboo mosaic virus (BaMV) promoter, particularly the PEBV promoter wherein the viral vector is a TRV vector, more particularly, the BaMV promoter wherein the viral vector is a PVX vector. In an additional embodiment, the sequence encoding the viral CP has been deleted in the viral vector of the invention, and a sequence encoding the viral CP, or a fragment thereof, is inserted under the control of a heterologous promoter, as just mentioned, particularly wherein the viral vector is a PVX vector. In a particular embodiment, the sequence encoding the viral CP has been deleted in the viral vector of the invention, and a sequence encoding the viral CP protein with a deletion of an N-terminal fragment, particularly the 29 amino acids at the N-terminal end of the viral CP protein, is inserted under the control of a heterologous promoter as just mentioned, particularly under the BaMV promoter.. In a particular embodiment, the sequence encoding the viral CP has been deleted in the viral vector of the invention, and a sequence encoding the viral CP protein with a deletion of an N-terminal fragment, particularly the 29 amino acids at the N-terminal end of the viral CP protein, is inserted under the control of a heterologous promoter as just mentioned, particularly under the BaMV promoter wherein the viral vector is a PVX vector. In another particular embodiment, the nucleic acid sequence of interest, in particular the nucleic acid sequence encoding or corresponding to S1, is inserted in the viral vector of the invention under the control of an endogenous promoter, particularly under the control of the viral CP promoter. In another particular embodiment, the nucleic acid sequence of interest, in particular the nucleic acid sequence encoding or corresponding to S1, is comprised in the viral vector of the invention under the control of an endogenous promoter, particularly under the control of the viral CP promoter, wherein the viral vector is a PVX vector, more particularly wherein the viral vector is as further defined in any of the five previous embodiments. In another particular embodiment, the nucleic acid sequence of interest, particularly the sequence encoding or corresponding to S1, is inserted in the viral vector of the invention under the control of a heterologous promoter, particularly under the control of the PEBV promoter or of the BaMV promoter. In another particular embodiment, the nucleic acid sequence of interest, particularly the sequence encoding or corresponding to S1, is comprised in the viral vector of the invention under the control of a heterologous promoter, particularly under the control of the PEBV promoter or of the BaMV promoter, wherein the viral vector is a TRV vector. In a further particular embodiment, the sequence encoding or corresponding to S1, is comprised in the viral vector of the invention under the control of the heterologous promoter PEBV promoter, particularly wherein the viral vector is a TRV vector. In a particular embodiment, the PEBV or BaMV promoter, as referred in any aspect of the invention, corresponds to or derives from the endogenous promoter of the CP protein in the PEBV, or the endogenous promoter of the CP protein in the BaMV, respectively. In another particular embodiment, the PEBV promoter as referred in any aspect of the invention, corresponds to or derives from the endogenous promoter of the CP protein of the PEBV. In another particular embodiment, the BaMV promoter, as referred in any aspect of the invention, rcorrespondsto the endogenous promoter of the CP protein of the BaMV. As well understood by a skilled person, the expression promoter is defined by reference to the protein encoded by the gene under the control of said expression promoter, i.e. to which the expression promoter is operatively linked. Thus, a CP promoter, as used herein, refers to a promoter that controls the expression of the gene encoding the viral CP, i.e. to which the gene encoding the viral CP protein is operatively linked.

Viral vectors can be RNA viral vectors or DNA viral vectors, depending on the virus from which they derive (RNA virus or DNA virus).

More particularly, RNA viruses can be single stranded (ss) or double stranded (ds). Single stranded RNA viruses can also be classified according to the sense or polarity of their RNA into negative-sense and positive-sense, or ambisense RNA viruses. Positive-sense viral RNA comprises the viral mRNA sequences that are translated in the cell, and thus can be immediately translated by the host cell. Negative-sense viral RNA is the RNA compelementary to that comprising the mRNA sequences that are translated in the cell, and thus must be converted to positive-sense RNA by an RNA-dependent RNA polymerase before translation. Ambisense RNA viruses resemble negative-sense RNA viruses, except they translate genes from their negative and positive strands. DNA viruese can be divided between those that are formed by two strands of DNA, called double-stranded DNA (dsDNA) virueses, and those that have one strand of DNA, called single-stranded DNA (ssDNA) viruses.

Double-stranded RNA (dsRNA) viruses are a polyphyletic group of viruses that have ds genomes made of ribonucleic acid. The ds genome is used as a template by the viral RNA-dependent RNA polymerase (RdRp) to transcribe a positive-strand RNA functioning as messenger RNA (mRNA) for the host cell's ribosomes, which translate it into viral proteins. The positive-strand RNA can also be replicated by the RdRp to create a new ds viral genome.

A viral vector that derives from an RNA virus is herein referred to as an RNA viral vector, and a viral vector derived from a DNA virus is herein referred to as a DNA viral vector. Concomitantly, an RNA viral vector that derives from a ssRNA virus, a ds RNA virus, a ss positive sense viral RNA, or a ss negative sense viral RNA, is herein referred to as a ssRNA viral vector, a dsRNA viral vector, a positive sense ssRNA viral vector, or a negative sense ssRNA viral vector, respectively. Similarly, a viral vector that derives from a ssDNA virus or a dsDNA virus, is herein referred to as a ssDNA viral vector or a dsDNA viral vector, respectively.

In a particular embodiment, the viral vector of the invention is selected from the list consisting of an RNA single stranded (ssRNA) viral vector, a dsRNA viral vector, a ssDNA viral vector and a dsDNA viral vector. In a particular embodiment, the ssRNA viral vector is a positive sense viral vector or a negative sense viral vector, particularly a positive sense viral vector. In a more particular embodiment, the viral vector of the first aspect of the invention is a positive sense ssRNA viral vector.

In a particular embodiment, the viral vector of the first aspect is a ssRNA viral vector selected from the list consisting of Potato Virus X (PVX) viral vector, Tobacco rattle virus (TRV) viral vector, Tobacco mosaic virus (TMV), tomato golden mosaic virus (TGMV), Cucumber mosaic virus (CMV), Barley stripe mosaic virus (BSMV), brome mosaic virus (BMV), and Foxtail mosaic virus (FoMV) .In a particular embodiment, the viral vector of the first aspect of the invention is an RNA viral vector selected from the list consisting of PVX viral vector, and TRV viral vector. In a more particular embodiment of the first aspect, the viral vector is a PVX viral vector. In another particular embodiment of the first aspect, the viral vector is a TRV viral vector. In another embodiment, the viral vector is a DNA viral vector selected from the list consisting of Bean yellow dwarf virus (BYDV), Wheat dwarf virus (WDV), and Cabbage leaf curl virus (CaLCuV).

The TRV virus is a member of the genus *Tobravirus* in the family of *Virgaviridae* and has a bipartite, positive sense ssRNA genome (RNA1 and RNA2). RNA1 and RNA2 are encapsidated separately into tubular, rigid, rod-shaped particles. The RNA1 encoded-proteins are sufficient for replication and movement of the virus within the host plant, while RNA2 encodes proteins for virion formation and nematode-mediated transmission. RNA1 encodes four open reading frames (ORFs) for proteins with predicted molecular weights of 134 and 194 kDa (replicase proteins), 29 kDa (movement protein), and 16 kDa (cysteine-rich protein, a silencing suppressor protein). RNA1 can replicate and move systemically without RNA2. RNA2 encodes three proteins (coat protein and two non-structural proteins, 29.4 kDa and 32.8 kDa). In a particular embodiment, the TRV vector, as referred herein, comprises the TRV RNA1 and RNA2 ssRNA genomes, or RNAs derived from the TRV RNA1 and RNA2 ssRNA genomes. In another particular embodiment, the viral vector of the invention is a TRV viral vector that comprises the RNA sequences derived from the RNA1 and RNA2 ssRNA genomes, wherein the heterologous nucleic acid sequence encoding or corresponding to S1, is comprised in the RNA sequence derived from the RNA2 ssRNA, particularly under the control of a heterologous promoter, more particularly under the control of the PEBV promoter. More particularly, the heterologous nucleic acid sequence encoding or corresponding to S1 is comprised between the first and the second ORF found in the TRV RNA2 ssRNA in the viral vector of the invention, particularly between the sequence encoding the viral CP protein and the sequence encoding the non-structural proteins of 29.4 kDa in the viral vector of the invention, particularly, wherein the viral vector of the invention comprises the RNA sequences derived from the RNA1 and RNA2

The term "region", in the context of the RNA sequence S1 as defined herein, refers to an RNA sequence that is a subunit of S1. Thus, said region is a short RNA sequence comprised within the RNA sequence S1, as defined herein, and with a length shorter than that of sequence S1, i.e. with a number of nucleotides lower than the number of nucleotides of S1. As well understood by a skilled person, the length of S1 is equal or higher the sum of the lengths of regions R1, R2, and R3 comprised in S1. When the length of S1 is higher than the sum of the lengths of regions R1, R2, and R3, S1 comprises RNA sequences or ribonucleotides in addition to those of regions R1, R2 and R3. In a particular embodiment, the length of S1 is equal to the sum of the lengths of regions R1, R2, and R3. In a particular embodiment, S1 consists of regions R1, R2, and R3.

As well understood by a skilled person, the nucleotides of the RNA sequence S1 are ribonucleotides. Thus, the nucleotides of regions R1, R2 and R3 consist of ribonucleotides. In a particular embodiment, the nucleotides referred herein as part of the RNA sequence S1, of region R1, R2, and/or R3 are ribonucleotides.

As well understood by a skilled person, S1 comprises in the 5'-end to the 3'-end direction, directly linked to each other, R1, R2 and R3. As well understood by a skilled person, "directly linked to each other" in the context of sequence S1, indicates that R1 is directly linked to R2 and R2 is directly linked to R3.

The term "directly linked", as used herein refers to the fact the 3' end nucleotide of an RNA region, or RNA sequence, is linked to the 5' end nucleotide of another RNA region, or RNA sequence, by a covalent bond, particularly by a phosphodiester bond. More specifically, the phosphodiester bond is formed between the 3'-OH of the 3' end nucleotide of one RNA region or RNA sequence, and the 5' phosphate group of the 5' end nucleotide of another RNA region or RNA sequence.

In a particular embodiment of the first aspect of the invention, the RNA sequence S1 has a length 50-400, 50-350, 50-300, 50-250, 50-200, 50-159, 50-150, 50-138, 50-134, 50-130, 50-120, 50-117, 50-113, 50-100, 50-96, 50-92, 50-90, 50-75, or 50-71 nucleotides, particularly, 50-113 nucleotides. In a particular embodiment of the first aspect of the invention, the RNA sequence S1 has a length of 53-400, 53-350, 53-300, 53-250, 53-200, 53-159, 53-158, 53-150, 53-138, 53-137, 53-130, 53-120, 53-117, 53-116, 53-100, 53-96, 53-95, 53-90, 53-75, or 53-74 nucleotides, particularly, 53-116 nucleotides. In a particular embodiment of the first aspect of the invention, the RNA sequence S1 has a length of 54-400, 54-350, 54-300, 54-250, 54-200, 54-159, 54-150, 54-138, 54-130, 54-120, 54-117, 54-100, 54-96, 54-90, or 54-75 nucleotides, particularly, 54-117 nucleotides. In another particular embodiment, the RNA sequence S1 has a length of 75-96, 75-117, 75-138, 75-159, or 75-180 nucleotides, particularly of 75-117 nucleotides. In another particular embodiment, the RNA sequence S1 has a length of 96-117, 96-138, 96-159, or 96-180 nucleotides, particularly of 96-117 nucleotides. In another particular embodiment, the RNA sequence S1 has a length of 117-138, 117-159, or 117-180 nucleotides, particularly of 117-138 nucleotides. In another particular embodiment, the RNA sequence S1 has a length of 138-159, or 138-180 nucleotides, particularly of 138-158 nucleotides. In another particular embodiment, of the first aspect of the invention, the RNA sequence S1 has a length of at least 50, at least 51, at least 52, at least 53, at least 54, at least at least 55, at least 70, at least 71, at least 72, at least 73, at least 74, at least 113, at least 112, at least 114, at least 115, at least 116, at least 117, at least 134, at least 136, at least 137, at least 138, at least 155, at least 156, at least 157, at least 158, at least 159, , at least 178, at least 178, at least 179, at least 180 nucleotides, particularly at least 54 nucleotides. In another particular embodiment, the RNA sequence S1 has a length of 50, 51, 52, 53, 54, 55, 70, 71, 72, 73, 74, 113, 112, 114, 115, 116, 117, 134, 135, 136, 137, 138, 155, 156, 157, 158, 159, 178, 178, 179, 180 nucleotides, particularly 54 nucleotides. In another particular embodiment, the RNA sequence S1 has a length of 54, 75, 96, 117, 138, 159 or 180 nucleotides, particularly of 117 nucleotides.

Ina another particular embodiment of the first aspect, the length of the sequence comprised in S1 consisting of regions R1, R2 and R3, as defined herein, has a length of 50-113, 53-116, or 54-117 nucleotides. Inn another particular embodiment of the first aspect, the length of the sequence comprised in S1 consisting of regions R1, R2and R3, as defined herein, has a length of 75-96, 75-117, 75-138, 75-159, or 75-180 nucleotides, particularly of 75-117 nucleotides. In another particular embodiment, the length of the sequence comprised in S1 consisting of regions R1, R2 and R3, as defined herein, is of 96-117, 96-138, 96-159, or 96-180 nucleotides, particularly of 96-117 nucleotides. In another particular embodiment, the length of the sequence comprised in S1 consisting of regions R1, R2 and R3, as defined herein, is of 117-138, 117-159, or 117-180 nucleotides, particularly of 117-138 nucleotides. In another particular embodiment, the length of the sequence comprised in S1 consisting of regions R1, R2 and R3, as defined herein, is of 138-159, or 138-180 nucleotides, particularly of 138-158 nucleotides. In another particular embodiment, the length of the sequence comprised in S1 consisting of regions R1, R2 and R3, as defined herein, is of 52, 73, 94, 115, 136, 157 or 178 nucleotides, particularly of 115 nucleotides. In another particular embodiment, the length of the sequence comprised in S1 consisting of regions R1, R2 and R3, as defined herein, is of 53, 74, 95, 116, 137, 158 or 179 nucleotides, particularly of 116 nucleotides. In a more particular embodiment, the length of the sequence comprised in S1 consisting of regions R1, R2 and R3, as defined herein, is of 54, 75, 96, 117, 138, 159 or 180 nucleotides, particularly of 117 nucleotides. In another particular embodiment, the length of the sequence comprised in S1 consisting of regions R1, R2 and R3, as defined herein, is of 54 nucleotides.

in another particular embodiment of the first aspect, the RNA sequence S1 consists of the regions R1, R2, and R3, and at least 1, at least 2, at least 3, at least 4, at least 5, at least 10, at least 15, at least 20, at least 30, at least 40, at least 50, at least 75, at least 100, at least 150, at least 200 at least 250, at least 300, at least 311, at least 346 nucleotides upstream region R1 in a 5' to 3'-end direction. In another particular embodiment of the first aspect, the RNA sequence S1 consists of the regions R1, R2, and R3, and 5-346, 5-311, 5-300, 5-250, 5-200, 5-150, 5-100, 5-50, 5-40, 5-30, 5-20, 5-15, or 5-10 nucleotides upstream region R1 in a 5' to 3'-end direction. In another particular embodiment of the first aspect, the RNA sequence S1 consists of the regions R1, R2, and R3, and at least 1, at least 2, at least 3, at least 4, at least 5, at least 10, at least 15, at least 20, at least 30, at least 40, at least 50, at least 75, at least 100, at least 150, at least 200 at least 250, at least 300, at least 311, at least 346 nucleotides downstream region R3 in a 5' to 3'-end direction. In another particular embodiment of the first aspect, the RNA sequence S1 consists of the regions R1, R2, and R3, and 5-346, 5-311, 5-300, 5-250, 5-200, 5-150, 5-100, 5-50, 5-40, 5-30, 10-20, 5-15, or 5-10 nucleotides downstream of region R3 in a 5' to 3'-end direction. In another particular embodiment, the RNA sequence S1 consists of the regions R1, R2, and R3, and at least 3, particularly at least 15 nucleotides upstream region R1 in a 5' to 3'-end direction, and at least 3, particularly at least 15 nucleotides downstream of region R3 in a 5' to 3'-end direction. In another particular embodiment, the RNA sequence S1 consists of the regions R1, R2, and R3, and 5-15, particularly at least 5-100 nucleotides upstream region R1 in a 5' to 3'-end direction, and 5-15, particularly 5-100 nucleotides downstream of region R3 in a 5' to 3'-end direction.

The term "nucleic acid", as used herein, relates to a deoxyribonucleotide or ribonucleotide polymer in either single or double stranded form and, unless otherwise limited, encompasses natural nucleotides and analogues of natural nucleotides that hybridize to nucleic acids in a manner similar to naturally occurring nucleotides. The term "nucleotide" includes, but is not limited to, a monomer that includes a base (such as a pyrimidine, purine or synthetic analogs thereof) linked to a sugar (such as ribose, deoxyribose or synthetic analogs thereof), or a base linked to an amino acid, as in a peptide nucleic acid (PNA). A nucleotide is one monomer in an oligonucleotide or in a polynucleotide. A "nucleotide sequence" or "nucleic acid sequence" refers to the sequence of bases in an oligonucleotide or in a polynucleotide.

The term "length" or "has a length of", as used herein in the context of an RNA sequence, refers to the number of nucleotides that said RNA sequence has. When used in the context of the length of an RNA duplex, it can be indicated as the number of nucleotides comprised in said duplex, o as the number of bp, i.e. nucleotides base paired to each other or pairs of nucleotides complementary to each other, in said duplex.

In a particular embodiment, the heterologous nucleotide sequence that encodes, or corresponds to the RNA sequence S1 in the viral vector of the invention is operatively linked to an expression promoter. Examples of suitable expression promoters include those conventionally used in molecular biology and known to the skilled person. In a particular embodiment of the first aspect, the expression promoter to which the heterologous nucleotide sequence that encodes, or corresponds to S1 is operatively linked in the viral vector of the invention, is selected from the list consisting of the PEBV promoter, the BaMV promoter, and the CP promoter, particularly the PVX CP promoter. In another particular embodiment of the first aspect, the expression promoter to which the heterologous nucleotide sequence that encodes, or corresponds to S1 is operatively linked in the viral vector of the invention, is selected from the list consisting of the endogenous promoter of the CP protein in the PEBV, the endogenous promoter of the CP protein in the BaMV, the endogenous promoter of the CP protein in the PVX, and the endogenous promoter of the CP protein in the viral vector of the invention. In another particular embodiment of the first aspect, the expression promoter to which the heterologous nucleotide sequence that encodes, or corresponds to S1 is operatively linked in the viral vector of the invention, is an expression promoter derived from an expression promoter selected from the list consisting of the endogenous promoter of the CP protein in the PEBV, the endogenous promoter of the CP protein in the BaMV, the endogenous promoter of the CP protein in the PVX, and the endogenous promoter of the CP protein of the viral vector of the invention.

In a particular embodiment of the first aspect, the heterologous sequence that encodes, or corresponds to S1 is operatively linked to the PEBV expression promoter, particularly to the expression promoter consisting of or derived from the endogenous CP promoter in the PEBV. In a particular embodiment of the first aspect, the viral vector of the invention is a TRV vector, and the heterologous sequence that encodes, or corresponds to S1 is operatively linked to the PEBV expression promoter, particularly to the expression promoter consisting of or derived from the endogenous CP promoter in the PEBV. In another particular embodiment of the first aspect, the heterologous sequence that encodes, or corresponds to S1 is operatively linked to the endogenous expression promoter of the CP protein in the viral vector of the invention, particularly wherein the viral vector is a PVX viral vector.

The expression "operatively linked to an expression promoter" as used herein in the context of a nucleotide sequence in the viral vector of the invention, indicates that said nucleotide is under the control of the expression promoter so that synthesis of the corresponding RNA sequence can take place from the sequence of the expression promoter. As well understood by a skilled person, the nucleotide sequence operatively linked to the expression promoter does not need to be directly linked to the corresponding promoter sequence and nucleotides can be comprised between the 3' end of the corresponding sequence of the expression promoter and the 5' end of the nucleotide sequence operatively linked to the expression promoter.

The term "heterologous sequence", as used herein refers to any nucleic acid sequence comprised in the viral vector of the invention that is not comprised in the viral DNA or RNA from which the viral vector derives. In other words, a heterologous sequence is a segment of nucleic acid within or attached to another nucleic acid molecule that is not found in association with the other molecule in nature. For example, a heterologous region of a nucleic acid construct could include a coding sequence flanked by sequences not found in association with the coding sequence in nature.

In a particular embodiment, the viral vector of the invention comprises, in addition to the heterologous nucleotide sequence that encodes, or corresponds to the RNA sequence S1, additional heterologous sequences. In a particular embodiment, said additional heterologous sequences comprise the sequence of a heterologous expression promoter. In a particular embodiment, the heterologous expression promoter is the PEBV expression promoter, or the BaMV expression promoter.

In a particular embodiment, the sum of the lengths of all the heterologous sequences comprised in the viral vector of the first aspect is equal to or less than 400, 375, 350, 325, 300, 275,250, 225, 220, 215, 210, 200, 190, 180, 170, 160, 159, 150, 140, 130, 120, 117, 110, 105, 100, 96, 95, 94, 93, 92, 91, 90, 89, 87, 86, 85, 82, 80, 75, 70, 65, 62, 60, 55, 54, 52, or 51 nucleotides, particularly equal to or less than 300 nucleotides. In another particular embodiment, the sum of the lengths of all the heterologous sequences comprised in the viral vector of the first aspect of the invention is equal to, or less than 250 nucleotides. In another particular embodiment, the sum of the lengths of all the heterologous sequences comprised in the viral vector of the first aspect of the invention is equal to, or less than 159 nucleotides. In another particular embodiment, the sum of the lengths of all the heterologous sequences comprised in the viral vector of the first aspect of the invention is equal to, or less than 150 nucleotides. In another particular embodiment, the sum of the lengths of all the heterologous sequences comprised in the viral vector of the first aspect of the invention is equal to, or less than 138 nucleotides. In another particular embodiment, the sum of the lengths of all the heterologous sequences comprised in the viral vector of the first aspect of the invention is equal to, or less than 117 nucleotides. In another particular embodiment, the sum of the lengths of all the heterologous sequences comprised in the viral vector of the first aspect of the invention is equal to, or less than 96 nucleotides. In another particular embodiment, the sum of the lengths of all the heterologous sequences comprised in the viral vector of the first aspect of the invention is equal to, or less than 75 nucleotides. In another particular embodiment, the sum of the lengths of all the heterologous sequences comprised in the viral vector of the first aspect of the invention is equal to, or less than 54 nucleotides. In another particular embdodiment the sum of the lengths of all the heterologous sequences comprised in the viral vector of the first aspect of the invention is of 50-400, 50-350, 50-300, 50-250, 50-200, 50-159, 50-150, 50-138, 50-134, 50-130, 50-120, 50-117, 50-113, 50-100, 50-96, 50-92, 50-90, 50-75, or 50-71 nucleotides, particularly, 50-113 nucleotides. In another particular embdodiment the sum of the lengths of all the heterologous sequences comprised in the viral vector of the first aspect of the invention is of 53-400, 53-350, 53-300, 53-250, 53-200, 53-159, 53-158, 53-150, 53-138, 53-137, 53-130, 53-120, 53-117, 53-116, 53-100, 53-96, 53-95, 53-90, 53-75, or 53-74 nucleotides, particularly, 53-116 nucleotides. In another particular embdodiment the sum of the lengths of all the heterologous sequences comprised in the viral vector of the first aspect of the invention is of 54-400, 54-350, 54-300, 54-250, 54-200, 54-159, 54-150, 54-138, 54-130, 54-120, 54-117, 54-100, 54-96, 54-90, or 54-75 nucleotides, particularly, 54-117 nucleotides. In another particular embodiment the sum of the lengths of all the heterologous sequences comprised in the viral vector of the first aspect of the invention is of 75-96, 75-117, 75-138, 75-159, or 75-180 nucleotides, particularly of 75-117 nucleotides. In another particular embodiment the sum of the lengths of all the heterologous sequences comprised in the viral vector of the first aspect of the invention is of 96-117, 96-138, 96-159, or 96-180 nucleotides, particularly of 96-117 nucleotides. In another particular embodiment the sum of the lengths of all the heterologous sequences comprised in the viral vector of the first aspect of the invention is of 117-138, 117-159, or 117-180 nucleotides, particularly of 117-138 nucleotides. In another particular embodiment the sum of the lengths of all the heterologous sequences comprised in the viral vector of the first aspect of the invention is of 138-159, or 138-180 nucleotides, particularly of 138-158 nucleotides. In another particular embodiment, In another particular embodiment the sum of the lengths of all the heterologous sequences comprised in the viral vector of the first aspect of the invention is of 50, 51, 52, 53, 54, 55, 70, 71, 72, 73, 74, 113, 112, 114, 115, 116, 117, 134, 135, 136, 137, 138, 155, 156, 157, 158, 159, 178, 178, 179, or 180 nucleotides, particularly 54 nucleotides. In another particular embodiment the sum of the lengths of all the heterologous sequences comprised in the viral vector of the first aspect of the invention is of 54, 75, 96, 117, 138, 159 or 180 nucleotides, particularly of 117 nucleotides.

The term "sRNA", or "small RNA", as used herein, refers to 20-24 nucleotide length RNAs that base-pair with their target mRNA to negatively regulate its expression. They are derived from double-stranded RNAs through the combined activity of several biogenesis and processing components. These sRNAs function by negatively regulating the expression of target genes. In eukaryotes, small RNAs can be classified into four groups based on their origins: microRNA (miRNA), small interfering RNA(siRNA), PiWi-interacting RNA (piRNA) and transfer RNA-derived small RNA (tsRNA). In plants, small RNAs are generally classified into miRNAs and siRNAs.

The term "siRNA", or "small interfering RNA", "short interfering RNA" or "silencing RNA", as used herein, refers to sRNA (20-24 nucleotide length RNAs that base-pair with their target mRNA to negatively regulate its expression) that result from the processing of dsRNA, that can be formed by the hybridization of sense and antisense transcripts, the folding back of an inverted-repeat sequence, the hybridization of unrelated RNA molecules with sequence complementarity, or by transcription or enzymes such as RNA-dependent RNA polymerases (RdRPs), which use single-stranded RNA as a template to generate long dsRNA. This dsRNA is further processed to form an siRNA duplex, with 2 nucleotides overhang at the 3'end of each strand of the duplex, and a core of 19 nucleotide base-paired region. The strand of the siRNA duplex that is complementary to the target RNA of the siRNA is referred to as the "guide strand" and the other strand of the siRNA duplex is referred to as the "passenger strand". Typically, one of the strands of the duplex (the guide strand) is sorted out into an ARGONAUTE (AGO) protein resulting in an RNA-induced silencing complex (RISC) that recognizes and silences complementary target RNAs through their endonucleolytic cleavage. The sequence of an siRNA generally provided to define the siRNA is the sequence complementary to its target sequence, i.e. the sequence of the guide strand of the siRNA. In other words, the term "sequence of a siRNA" or "siRNA sequence", generally corresponds to the sequence of the corresponding siRNA guide strand. In a particular embodiment, the term "siRNA sequence" or "sequence of an siRNA", as used herein, refers to the sequence of the siRNA that is complementary to the target site of the siRNA, i.e. to the sequence of the corresponding siRNA guide strand.

The term "miRNA" or "micoRNA", as used herein, refers to sRNA (20-24 nucleotide length RNAs that base-pair with their target mRNA to negatively regulate its expression) that result from the processing of a primary transcript of miRNA (pri-miRNAs). Pri-miRNAs are capped, spliced and polyadenylated, and harbor a characteristic foldback (or hairpin) structure that also varies in length (from 50 to 900 nt). MiRNA foldbacks have a common structure consisting of a ~14-17 bp basal stem (BS), a miRNA/miRNA* duplex, and a distal stem-loop (DSL) region variable in size and shape and flanked by single-stranded RNA (ssRNA) segments. Processing of most pri-miRNAs occurs through a "base-to-loop" mechanism in which a nuclear RNase III DICER-LIKE (DCL) protein, commonly DCL1 associated with its accessory proteins SERRATE (SE) and HYPONASTIC LEAVES1 (HYL1) first cleaves at the BS of the foldback to release a shorter precursor miRNA (pre-miRNA). Next, a second cleavage at a distance equivalent to the length of the mature miRNA (20-24 nucleotides) from the first cut releases the miRNA/miRNA* duplex including two-nucleotide 3' overhangs that are 2'-O-methylated by the methyltransferase HUA ENHANCER1 (HEN1). Alternatively, for loop-to-base processed precursors, the DCL protein, commonly DCL1, produces a first cut that releases the terminal loop and then continues towards the base of the precursor to release the miRNA/miRNA* duplex. Typically, one of the strands of the duplex (the guide strand) is sorted out into an ARGONAUTE (AGO) protein (AGO1 for the majority of plant miRNAs) resulting in an RNA-induced silencing complex (RISC) that recognizes and silences complementary target RNAs mostly through their endonucleolytic cleavage. The strand of the miRNA/miRNA* duplex that is complementary to the target mRNA sequence is referred to as the guide strand, or the mature miRNA, and the other strand of the miRNA/miRNA* duplex is referred to as the passenger strand, which is generally subject to degradation once the guide strand is sorted out in the AGO protein. Commonly, the sequence of a miRNA given to define a miRNA is the sequence complementary to its target sequence, i.e. the sequence of the mature miRNA or of the corresponding miRNA guide strand. In other words, the term "sequence of a miRNA" or "miRNA sequence", generally corresponds to the sequence of the mature miRNA. In a particular embodiment, the term "miRNA sequence" or "sequence of an miRNA", as used herein, refers to the sequence of the miRNA that is complementary to the target site of the miRNA, i.e. to the sequence of the mature miRNA or of the miRNA guide strand.

In a particular embodiment of the first aspect of the invention, the sRNA whose target sequence corresponds to that of R1 is a miRNA or an siRNA. In a particular embodiment of the first aspect of the invention, the sRNA of the invention is a miRNA. In another particular embodiment of the first aspect, the sRNA of the invention is a siRNA. In a more particular embodiment, the sRNA of the invention has a length of 22 or 21 nucleotides, particularly 22 nucleotides. In another particular embodiment of the first aspect, the sRNA of the invention is miRNA of 22 or 21 nucleotides, particularly of 22 nucleotides. In another particular embodiment of the first aspect, the sRNA of the invention is a siRNA of 22 or 21 nucleotides, particularly of 22 nucleotides.

In a particular embodiment, region R1 consists of the target site of an sRNA, particularly a miRNA or a siRNA. In a particular embodiment, region R1 consists of the target site of miRNA. In another particular embodiment, region R1 consists of the target site of siRNA

In a particular embodiment, region R1 has a length of 21 or 22 nucleotides, particularly 22 nucleotides. In another particular embodiment, region R1 consists of the target site of a miRNA or a siRNA and has a length of has a length of 21 or 22 nucleotides, particularly 22 nucleotides. More particularly, region R1 consists of the target site of a miRNA and has a length of has a length of 21 or 22 nucleotides, particularly 22 nucleotides.

The term "target site", or "TS" as used herein in the context of an sRNA, refers to the RNA sequence complementary to the sequence of the corresponding sRNA in the target RNA molecule of the sRNA. Thus, the sequence of an sRNA target sites is complementary to the sequence of the corresponding sRNA. Complementarity between the sRNA sequence and the sRNA target site sequence does not need to be perfect. In case complementarity is not perfect between both sequences, it is preferred that nucleotides in positions 3-12, 2-13, 1-14 of the sRNA target site in a 5'- to 3'-end direction form a duplex, generally with the same number of nucleotides of the sRNA, wherein said duplex is preferably perfectly matched, or comprises no more than 1 or 2 mismatches. In addition, commonly, the target site of an sRNA has the same length as the corresponding sRNA. Thus, in a particular embodiment of the first aspect, the target site of the sRNA of the invention has the same length as the sRNA of the invention, more particularly R1 has the same length as the sRNA of the invention.

The term "base pairing" or "complementarity" as used herein, refers to the interaction between at least two nucleotides by hydrogen bonds. Thus, at least two nucleotides that interact by hydrogen bonds are understood to "base pair" or "to be complementary" between each other. Base pairing between ribonucleotides, as used herein, includes Watson-Crick base pairing (A-U and C-G ribonucleotide base pairing) and wobble base pairing (particularly G-U ribonucleotide base pairing). In a particular embodiment, base pairing as used herein refers to base pairing between two RNA molecules or two regions within an RNA molecule. When several nucleotides in a sequence base pair with several nucleotides of another sequence, said sequences are commonly understood to base pair with each other or to be complementary to each other. Base pairing between two sequences can be complete or perfect, i.e. all the nucleotides within a sequence base pair with all the nucleotides of another sequence, or partial or imperfect, wherein several but not all the nucleotides of one sequence base pair with nucleotides in the other sequence. When at least two contiguous nucleotides in an RNA sequence base pair with nucleotides in another RNA sequence, the resulting structure motif is described as a stem, a duplex or a helix. Those nucleotides that do not base pair within a duplex or stem structure are generally considered to form mismatches or bulges within the duplex. As well known by an expert in the field, a mismatch occurs when two complementary nucleotide pairs are separated by a single non complementary pair of nucleotides in an RNA duplex. A bulge is the result of at least one unpaired nucleotide in only one strand of duplex. Base pairing, as well as the degree of base pairing, between two nucleotide sequences can be easily predicted, following the Watson and Crick or wobble base paring rules indicated above. Methods to determine the base pairing that can occur between two RNA sequences are well-known by an expert in the field and include the use of computer programs such as RNASoft, or PairFold.

In a particular embodiment, the sequence of sRNA target site corresponding to R1 is that of the natural target site of the sRNA of the invention. The term "natural target site", as used herein, refers to the target site of the corresponding sRNA that is comprised in the mRNA target of said sRNA in the plant that endogenously expresses the sRNA. In another particular embodiment, the sequence of the sRNA target site corresponding to R1 is not identical to that of the natural target site of the sRNA of the invention, i.e. it is an artificial target site of said sRNA.

In a particular embodiment, the sequence of the sRNA target site corresponding to R1 is complementary to the sequence of the sRNA of the invention. In another particular embodiment, the sequence of the sRNA target site corresponding to R1 is perfectly complementary to the sequence of the sRNA of the invention. In another particular embodiment, the sequence of the sRNA target site corresponding to R1 is partially complementary to the sequence of the sRNA of the invention. In another particular embodiment, the sequence of the sRNA target site corresponding to R1 is partially complementary to the sequence of the sRNA of the invention, wherein nucleotides between positions 3-12, 2-13, 1-14, particularly between positions 2-13 of the sRNA target site in a 5' to 3'-end direction form a duplex with the sRNA of the invention (herein referred to as sRNA/TS duplex), wherein the duplex comprises no more than 2 mismatches. In another particular embodiment, the sequence of the sRNA target site corresponding to R1 is partially complementary to the sequence of the sRNA of the invention, wherein nucleotides between positions 3-12, 2-13, 1-14, particularly between positions 2-13 of the sRNA target site in a 5' to 3'-end direction form a duplex with the sRNA of the invention (i.e. the sRNA/TS duplex), wherein the sRNA/TS duplex comprises no more than 1 mismatch. In another particular embodiment, the sequence of the sRNA target site corresponding to R1 is partially complementary to the sequence of the sRNA of the invention, wherein nucleotides between positions 3-12, 2-13, 1-14, particularly between positions 2-13 of the sRNA target site in a 5' to 3'-end direction form a perfectly base paired duplex with the sRNA of the invention (i.e. the sRNA/TS duplex), i.e. the TS sequence and the sRNA sequence forming the sRNA/Rs duplex are perfectly complementary.

Thus, in a particular embodiment, region R1 is complementary to the sRNA of the invention. In another particular embodiment, region R1 is perfectly complementary to the sRNA of the invention. In another particular embodiment, region R1 is partially complementary to the sRNA of the invention. In another particular embodiment, region R1 is partially complementary to the sRNA of the invention, wherein nucleotides between positions 3-12, 2-13, 1-14, particularly between positions 2-13 of region R1 in a 5'- to 3'-end direction form a duplex with the sRNA of the invention (herein referred to as sRNA/R1 duplex), wherein the sRNA/R1 duplex comprises no more than 2 mismatches. In another particular embodiment of the first aspect, region R1 is partially complementary to the sRNA of the invention, wherein nucleotides between positions 3-12, 2-13, 1-14, particularly between positions 2-13 of region R1 in a 5'- to 3'-end direction form a duplex with the sRNA of the invention (herein referred to as sRNA/R1 duplex), wherein the sRNA/R1 duplex comprises no more than 1 mismatch. In another particular embodiment, region R1 is partially complementary to the sequence of the sRNA of the invention, wherein nucleotides between positions 3-12, 2-13, 1-14, particularly between positions 2-13 of region R1 in a 5' to 3'-end direction form a perfectly base paired duplex with the sRNA of the invention (i.e. the sRNA/R1 duplex), i.e. region R1 and the sRNA sequence forming the sRNA/R1 duplex are perfectly complementary.

In a particular embodiment, region R1 consists of the target site of an sRNA, particularly a miRNA or a siRNA, with a cleavage site between positions 10 and 11 in a 3'- to 5'-end direction. In another particular embodiment, region R1 consists of the target site of a miRNA with a cleavage site between positions 10 and 11 in a 3'- to 5'-end direction. In another particular embodiment, region R1 consists of the target site of a siRNA with a cleavage site between positions 10 and 11 in a 3' to 5'-end direction.

The term" cleavage site", as used herein, refers to the position within an sRNA target site or target mRNA in which the ribonuclease complex RISC comprising the sRNA complementary to said sRNA target site or target mRNA cleaves that target site or target mRNA. Thus, when the sRNA is an siRNA, the cleavage site in its target site corresponds to the position within siRNA target site that is cleaved by the RISC complex which comprises the corresponding siRNA guide strand. When the sRNA is an miRNA, the cleavage site in its target site corresponds to the position within the miRNA target site that is cleaved by the RISC complex which comprises the corresponding miRNA guide strand.

In a particular embodiment, the sRNA of the invention is a miRNA selected from the list consisting of AtMIR173, NbMIR482a, NbMIR6019a, SIMIR482b, SlmiR6020. In a more particular embodiment, the sRNA of the invention is an sRNA, particularly a 22 nucleotides long sRNA, endogenously expressed in the plant of the invention, wherein the plant of the invention is as defined below.

The term "AMIR173", as used herein refers to the miR173 endogenously expressed in the plant *Arabidopsis thaliana* with sequence corresponding to SEQ ID NO. 7 (UUCGCUUGCAGAGAGAAAUCAC).

The term "NbMIR482a", as used herein, refers to the miR482a endogenously expressed in the plant *Nicotiana benthamiana,* with sequence SEQ ID NO. 8 (UUCCCGACUCCCCCCAUACCAC)

The term "NbMIR6019a", as used herein, refers to the miR619a endogenously expressed in the plant *Nicotiana benthamiana,* with sequence SEQ ID NO. 9 (UACAGGUGACUUGUAAAUGUUU).

The term "SIMIR482b" as used herein, refers to the miR482b endogenously expressed in the plant *Solanum lycopersicum,* with sequence SEQ ID NO. 10 (UCUUGCCUACACCGCCCAUGCC).

The term SIMIR6020 as used herein, refers to the miR6020 endogenously expressed in the plant *Solanum lycopersicum,* with sequence SEQ ID NO. 31 (UAAAUGUUUUCCGAGUAUCUUU).

In a particular embodiment, the target site of the sRNA AtMIR173 has sequence SEQ ID NO. 2 (GUGAUUUUUCUCUACAAGCGAA). Thus, in another particular embodiment R1 consists of SEQ ID NO. 2, particularly wherein the plant of the invention is *Arabidopsis sp,* more particularly *Arabidopsis thaliana.* In a particular embodiment, when region R1 has SEQ ID NO. 2, the sRNA of the invention is AtMIR173.

In another particular embodiment, the target site of the sRNA NbMIR482a has sequence SEQ ID NO. 3 (GUGGUAUGGGGGGAGUCGGGAA). Thus, in another particular embodiment R1 consists of SEQ ID NO. 3, particularly wherein the plant of the invention is *Nicotiana sp,* more particularly *Nicotiana benthamiana.* In a particular embodiment, when region R1 has SEQ ID NO. 3, the sRNA of the invention is NbMIR482a.

In another particular embodiment, the target site of the sRNA NbMIR6019a has sequence SEQ ID NO. 4 (AAACAUUUACAAGUCACCUGUA). Thus, in another particular embodiment R1 consists of SEQ ID NO. 4, particularly wherein the plant of the invention is *Nicotiana sp,* more particularly *Nicotiana benthamiana.* In a particular embodiment, when region R1 has SEQ ID NO. 4, the sRNA of the invention is NbMIR6019a.

In another particular embodiment, the target site of the sRNA SIMIR482b has sequence SEQ ID NO. 5 (GGCAUGGGCGGUGUAGGCAAGA). Thus, in another particular embodiment R1 consists of SEQ ID NO. 5, particularly wherein the plant of the invention is *Solanum sp,* particularly *Solanum lycopersicum.* In a particular embodiment, when region R1 has SEQ ID NO. 5, the sRNA of the invention is SIMIR482b.

In another particular embodiment, the target site of the sRNA SlmiR6020 has sequence SEQ ID NO. 6 (AAAGAUACUCGGAAAACAUUUA). Thus, in another particular embodiment R1 consists of SEQ ID NO. 6, particularly wherein the plant of the invention is *Solanum sp,* more particularly *Solanum lycopersicum.* In a particular embodiment, when region R1 has SEQ ID NO. 6, the sRNA of the invention is SlmiR6020.

In a particular embodiment, the RNA sequence S1 corresponds to the RNA sequence of a functional syn-tasiRNA precursor. In another particular embodiment, the RNA sequence S1 corresponds to the RNA sequence upstream the poly-A tail of a functional syn-tasiRNA precursor. In another particular embodiment, the RNA sequence within the RNA sequence S1 consisting of regions R1, R2 and R3 corresponds to the RNA sequence of a functional syn-tasiRNA precursor. In another particular embodiment, the RNA sequence within the RNA sequence S1 consisting of regions R1, R2 and R3 corresponds to the RNA sequence upstream the poly-A tail of a functional syn-tasiRNA precursor. In another particular embodiment, the RNA sequence S1 consists of regions R1, R2, and R3 directly linked to each other as defined in formula (i), and corresponds to the RNA sequence of a functional syn-tasiRNA precursor. In another particular embodiment, the RNA sequence S1 consists of regions R1, R2, and R3 directly linked to each other as defined in formula (i), and corresponds to the RNA sequence upstream the poly-A tail of a functional syn-tasiRNA precursor. The term "tasi-RNA", ""ta-siRNA" or "Trans-acting siRNA", as used herein, refers to a class of small interfering RNA (siRNA) that repress gene expression through post-transcriptional gene silencing in plants. Precursor transcripts from TAS loci are polyadenylated and cleaved by a specific miRNA/AGO complex. One of the cleavage products is converted by RDR6 to dsRNA, which is processed by a DCL protein, commonly DCL4 into phased tasiRNA duplexes in 21-nt register with the miRNA cleavage site. TasiRNA guide strands are incorporated into AGO1 to direct silencing of one or multiple transcripts at one or multiple sites.

The term "syn-tasiRNA", "synthetic tasiRNA", "artificial tasi-RNA" or "atasiRNA", as used herein refer to the siRNAs expressed from transgenes including engineered TAS precursors in which a region corresponding to one or various endogenous tasiRNAs is substituted by a fragment containing one or multiple atasiRNA/syn-tasiRNA sequences.

The term "functional syn-tasiRNA precursor", as used herein refers to an RNA transcript that comprises siRNA sequences, particularly syn-tasiRNA sequences, that is processed as a TAS precursor transcript, particularly in a plant cell, even more particularly in a cell of the plant of the invention. Particularly, the functional syn-tasiRNA precursor comprises at least one si-RNA sequence, particularly at least one syn-tasiRNA sequence, it is cleaved by a specific miRNA/AGO complex, one of the cleavage products is converted by RDR6 to dsRNA, which is then processed by a DCL protein, particularly a DCL4 protein into phased siRNA duplexes, particularly tasiRNA duplexes, in 21-nt register with the miRNA cleavage site, particularly in a plant cell, more particularly in the cell of the plant of the invention. Thus, in a more particular embodiment, the functional syn-tasiRNA precursor is suitable for obtaining syn-tasiRNAs, particularly within a plant cell, more particularly within a cell of the plant of the invention. In another particular embodiment, the functional syn-tasiRNA precursor is suitable for DCL-mediated obtention of syn-tasiRNAs, particularly within a plant cell, more particularly within a cell of the plant of the invention. In another particular embodiment, the functional syn-tasiRNA precursor is suitable for DCL4-mediated obtention of syn-tasiRNAs, particularly within a plant cell, more particularly within a cell of the plant of the invention. In another particular embodiment, the functional syn-tasiRNA precursor comprises the sequences sufficient for obtaining syn-tasiRNAs, particularly when expressed in a plant cell, more particularly when expressed in a cell of the plant of the invention. In another particular embodiment, the functional syn-tasiRNA precursor comprises the sequences sufficient for DCL-mediated obtention of syn-tasiRNAs, particularly when expressed in a plant cell, more particularly when expressed in a cell of the plant of the invention. In another particular embodiment, the functional syn-tasiRNA precursor comprises the sequences sufficient for DCL4-mediated obtention of syn-tasiRNAs, particularly when expressed in a plant cell, more particularly when expressed in a cell of the plant of the invention. Methods to determine if an RNA sequence corresponds to that of functional syn-tasiRNA precursor include those disclosed in the examples below. For instance, the RNA sequence being analyzed can be incorporated into a pMDC32 plasmid to be expressed in a plant by agroinfiltration. The expression of the corresponding syn-tasiRNAs in the plant can then be determined by RT-PCR. In case syn-tasiRNAs are detected in the plant, it can be considered that the RNA sequence corresponds to that of a functional syn-tasiRNA precursor.

As indicated in the first aspect of the invention, region R2 consists of an RNA sequence of 11 ribonucleotides. In a particular embodiment, region R2 is a sequence spacer of 11 nucleotides, particularly a sequence spacer in the RNA sequence S1. The term "sequence spacer", as used herein, refers to a non-coding sequence that simply ensures that a certain distance is maintained between two sequences or regions of a polymer. In a particular embodiment, region R2 consists of an RNA sequence of 11 ribonucleotides that is single stranded in the secondary structure of sequence S1. Methods to determine the secondary structure of an RNA are well known by an expert in the field and include the use of bioinformatic tools such as RNAfold or mFOLD that predict the secondary structure of RNA sequences, or laboratory techniques such as Nuclear magnetic resonance (NMR) or Cryo-EM.

Region R2 provides a 21 nucleotides distance between nucleotide 10 of region R1 in a 3'- to 5'-end direction, and the 5'-end nucleotide of region R3. The said 21 nucleotide distance corresponds to the distance between the cleavage site in region R1 by the sRNA of the invention and the 5'end of region R3, allowing a DCL protein, particularly DL4, in a plant cell to release the siRNAs comprised in region R3, particularly when the RNA sequence S1 is expressed in the plant cell. Thus, region R2 allows the correct processing of the RNA sequence S1 expressed in a cell by the DCL protein, particularly the DCL4 protein, for the release of the siRNAs whose sequence is comprised in region R3. Therefore, methods to determine if an 11 ribonucleotides sequence is a sequence spacer, particularly in the RNA sequence S1 located between region R1 and region R3, as region R2, include determining if an RNA sequence S1 comprising said 11 ribonucleotides sequence as region R2 is a functional syn-tasi precursor. Examples of said methods are provided in the definition of "functional syn-tasi precursor".

In a particular embodiment, region R2 consists of the RNA sequence SEQ ID NO. 1 (UAGACCAUUUA) or a variant thereof at least 60%, at least 65%, at least 70%, at least 75%, at least 80%, at least 85%, at least 90% identical to SEQ ID NO.1.

The term "RNA sequence variants" or "RNA variants" or "variants" in the context of RNA sequences, are well understood by those of skill in the art and can involve nucleotide sequence modifications. For example, nucleotide sequence modifications typically fall into one or more of three classes: substitutional, insertional, or deletional variants.

In the present invention the term "identical" or "identity" refers to the percentage of nucleotides that are identical in the two sequences when the sequences are optimally aligned. If, in the optimal alignment, a position in a first sequence is occupied by the same nucleotide residue as the corresponding position in the second sequence, the sequences exhibit identity with respect to that position. The percentage of identity determines the number of identical residues over a defined length in a given alignment. Thus, the level of identity between two sequences or ("percent sequence identity") is measured as a ratio of the number of identical positions shared by the sequences with respect to the number of positions compared (i.e., percent sequence identity = (number of identical positions/total number of positions compared) x 100). A gap, i.e., a position in an alignment where a residue is present in one sequence but not in the other, is regarded as a position with non-identical residues and is counted as a compared position.

As an illustration, an RNA region having a nucleotide sequence being at least, for example, 75% identical to a reference RNA sequence is intended that the nucleic sequence of the RNA region is identical to the reference sequence except that the RNA sequence may include up to one nucleic acid alteration per each 4 nucleic acids of the reference sequence. In other words, to obtain an RNA sequence having a nucleic acid sequence of at least 75% identical to a reference nucleic acid sequence, up to 25% of the nucleic acid residues in the reference sequence may be deleted or substituted with another nucleic acid, or a number of nucleic acids up to 25% of the total nucleic acid residues in the reference sequence may be inserted into the reference sequence. These alterations of the reference sequence may occur at the 5' or 3' end terminal positions of the reference sequence or anywhere between those terminal positions, interspersed either individually among residues in the reference sequence or in one or more contiguous groups within the reference sequence.

A number of mathematical algorithms for rapidly obtaining the optimal alignment and calculating identity between two or more sequences are known and incorporated into a number of available software programs. For purposes of the present invention, the sequence identity between two nucleic acid sequences is preferably determined using algorithms based on global alignment, such as the Needleman-Wunsch algorithm (Needleman and Wunsch, 1970, J. Mol. Biol. 48: 443-453), preferably implemented in the Needle program of the EMBOSS package (EMBOSS: The European Molecular Biology Open Software Suite, Rice et al., 2000, Trends Genet. 16: 276-277); or the BLAST Global Alignment tool (Altschul et al., "Basic local alignment search tool", 1990, J. Mol. Biol, v. 215, pages 403-410), using default settings. Local alignment also can be used when the sequences being compared are substantially the same length.

In a particular embodiment, the sequence variant of SEQ ID NO. 1 contains up to 1, 2, 3, 4, 5, 6, 7, 8, 9, 10, particularly up to 4 ribonucleotide changes as compared to SEQ ID NO.1. In a particular embodiment, region R2 with SEQ ID NO. 1 or a variant thereof as defined herein, is a sequence spacer of 11 nucleotides, particularly a sequence spacer of 11 nucleotides in the RNA sequence S1. Methods to determine if an 11 ribonucleotides sequence is a sequence spacer, particularly in the RNA sequence S1 located between regions R1 and R3, as region R2, have been provided above.

In a particular embodiment, region R3 consists of the RNA sequence of one siRNA. In another particular embodiment, region R3 consists of the RNA sequence of more than one siRNA, wherein the siRNA sequences are directly linked to each other. In another particular embodiment, region R3 consists of the RNA sequence of more than one siRNA, wherein the one or more siRNA sequences are directly linked to each other. In another particular embodiment, region R3 consists of the RNA sequence of at least 2, at least 3, at least 4, at least 5, at least 6, at least 7, at least 8, at least 9, or at least 10 siRNAs, particularly of at least 4 siRNAs, wherein the siRNA sequences are directly linked to each other. In another particular embodiment, region R3 consists of the RNA sequence of at least 2, at least 3, at least 4, at least 5, at least 6, at least 7, at least 8, at least 9, or at least 10 siRNAs, particularly of at least 4 siRNAs, wherein the at least 2, at least 3, at least 4, at least 5, at least 6, at least 7, at least 8, at least 9, or at least 10 siRNA sequences, particularly the at least 4 siRNA sequences are directly linked to each other. In another particular embodiment, region R3 consists of the RNA sequence of 2, 3, 4, 5, 6, 7, 8, 9, or 10 siRNAs, particularly of 4 siRNAs, wherein the siRNA sequences are directly linked to each other. In another particular embodiment, region R3 consists of the RNA sequence of 2, 3, 4, 5, 6, 7, 8, 9, or 10 siRNAs, particularly of 4 siRNAs, wherein the 2, 3, 4, 5, 6, 7, 8, 9, or 10 siRNA sequences, particularly the 4 siRNA sequences, are directly linked to each other.

The term "siRNA" has been defined above in the context of region R1 and applies to the siRNAs whose sequence is comprised in region R3. As well understood by a skilled person, the siRNA sequences comprised in R3 are the sequences of the guide strand of the corresponding siRNAs. Thus in a particular embodiment, the siRNA sequences comprised in R3 are the sequences of siRNA guide strands. In a particular embodiment, the siRNA sequence comprised in R3 is an siRNA guide strand sequence. The one or more siRNAs whose sequences are comprised in R3 are herein referred to as the siRNAs of region R3. Concomitantly, the siRNAs whose sequences are comprised in R3 are herein referred as the siRNAs of region R3. If region R3 consists of the sequence of only one siRNA, said siRNA is herein referred as the siRNA of region R3. In other words, the siRNAs of region R3 are the siRNAs whose sequences directly linked to each other correspond to the sequence of region R3.

In a particular embodiment, the sequence of the siRNAs of region R3 have a length of 21 nucleotides. In another particular embodiment, the siRNAs whose sequence is comprised in region R3 have a length of 21 nucleotides. In another particular embodiment, the siRNAs of region R3 have a length of 21 nucleotides.

In a particular embodiment, the one or more siRNAs of region R3 target one or more genes in the genome of one or more pathogens of a plant.

In a particular embodiment, region R3 consists of the RNA sequence of one siRNA that targets one gene in the genome of a plant pathogen. In another particular embodiment, region R3 consists of the RNA sequence of at least 2, at least 3, at least 4, at least 5, at least 6, at least 7, at least 8, at least 9, or at least 10 siRNAs, particularly of at least 4 siRNAs, wherein the siRNA sequences of region R3 are directly linked to each other, and the siRNAs of region R3 target one or more genes in the genome of one or more plant pathogens. In another particular embodiment, region R3 consists of the RNA sequence of at least 2, at least 3, at least 4, at least 5, at least 6, at least 7, at least 8, at least 9, or at least 10 siRNAs, particularly of at least 4 siRNAs, wherein the siRNA sequences are directly linked to each other, and the at least 2, at least 3, at least 4, at least 5, at least 6, at least 7, at least 8, at least 9, or at least 10 siRNAs, particularly the at least 4 siRNAs target one or more genes in the genome of one or more plant pathogens. In another particular embodiment, region R3 consists of the RNA sequence of at least 2, at least 3, at least 4, at least 5, at least 6, at least 7, at least 8, at least 9, or at least 10 siRNAs, particularly of at least 4 siRNAs, wherein the siRNA sequences of the at least 2, at least 3, at least 4, at least 5, at least 6, at least 7, at least 8, at least 9, or at least 10 siRNAs, particularly of the at least 4 siRNAs are directly linked to each other and correspond to sequences of siRNAs that target one or more genes in the genome of one or more plant pathogens.

In another particular embodiment, region R3 consists of the RNA sequence of at least 2, at least 3, at least 4, at least 5, at least 6, at least 7, at least 8, at least 9, or at least 10 siRNAs, particularly of at least 4 siRNAs, wherein the siRNA sequences of region R3 are directly linked to each other, and the siRNAs of region R3 target one or more genes in the genome of one plant pathogen. In another particular embodiment, region R3 consists of the RNA sequence of at least 2, at least 3, at least 4, at least 5, at least 6, at least 7, at least 8, at least 9, or at least 10 siRNAs, particularly of at least 4 siRNAs, wherein the siRNA sequences of region R3 are directly linked to each other, and the siRNAs of region R3 target one or more genes in the genome of at least 2 plant pathogens.

In another particular embodiment, region R3 consists of the RNA sequence of at least 2, at least 3, at least 4, at least 5, at least 6, at least 7, at least 8, at least 9, or at least 10 siRNAs, particularly of at least 4 siRNAs, wherein the siRNA sequences of region R3 are directly linked to each other, and the siRNAs of region R3 target one gene in the genome of one plant pathogen.

In another particular embodiment, region R3 consists of the RNA sequence of at least 2, at least 3, at least 4, at least 5, at least 6, at least 7, at least 8, at least 9, or at least 10 siRNAs, particularly of at least 4 siRNAs, wherein the siRNA sequences of region R3 are directly linked to each other, and the siRNAs of region R3 target at least 2 genes in the genome of one plant pathogen.

In another particular embodiment, region R3 consists of the RNA sequence of at least 2, at least 3, at least 4, at least 5, at least 6, at least 7, at least 8, at least 9, or at least 10 siRNAs, particularly of at least 4 siRNAs, wherein the siRNA sequences of region R3 are directly linked to each other, and the siRNAs of region R3 target at least 2 genes in the genome of at least 2 plant pathogens.

The term "plant pathogen", as used herein, refers to organisms that can infect a plant, referred to as the host of the plant pathogen, and induces a disease in the host plant, at least in an organ of the host plant, including the leaf, stem, root, vascular system, flower and/or the fruit of the host plant.

In a particular embodiment, the one or more plant pathogens are selected from the list consisting of of viruses, viroids, bacteria, fungi, oomycetes, insects, nematodes and combinations thereof. In another particular embodiment, the one or more plant pathogens are selected from the list consisting of of viruses, viroids, bacteria, fungi, insects, nematodes and combinations thereof.

In a particular embodiment, the plant pathogen viruses are selected from the group consisting of Tomato spotted wilt virus (TSWV), Tomato brown rugose fruit virus (ToBRFV), Tomato leaf curl new delhi virus (ToLCNDV), Tomato mosaic virus (ToMV), Cucumber mosaic virus (CMV), Turnip mosaic virus (TuMV), Tomato yellow leaf curl virus (TYLCV), Potato virus Y (PVY), Cauliflower mosaic virus (CaMV), African cassava mosaic virus (ACMV), Plum pox virus (PPV), Brome mosaic virus (BMV), Potato virus X (PVX), Tobacco mosaic virus (TMV), Alfalfa mosaic virus (AMV), Citrus tristeza virus (CTV) and combinations thereof. In a particular embodiment, the viruses are selected from Tomato Spotted Wilt Virus (TSWV), Tomato brown rugose fruit virus (ToBRFV), Tomato leaf curl new delhi virus (ToLCNDV), and combinations thereof. In a more particular embodiment, the plant pathogen viruses are selected from the group consisting of Tomato spotted wilt virus (TSWV), Tomato brown rugose fruit virus (ToBRFV), Tomato leaf curl new delhi virus (ToLCNDV), and combinations thereof, particularly, is the Tomato spotted wilt virus (TSWV).

In another embodiment, the plant pathogen viruses are selected from the viruses disclosed in Barathi et al., (2023), Recent trends and advances of RNA interference (RNAi) to improve agricultural crops and enhance their resilience to biotic and abiotic stresses. Plant Physiology and Biochemistry 194:600-618, particularly from the list of viruses disclosed in table 5 of Barathi et al. (2023) *supra.*

In a particular embodiment, the one or more plant pathogens are viruses, particularly, are viruses selected from the list consisting of Tomato spotted wilt virus (TSWV), Tomato brown rugose fruit virus (ToBRFV), Tomato leaf curl new delhi virus (ToLCNDV), and combinations thereof. In a more particular embodiment, the one or more plant pathogens include the Tomato spotted wilt virus (TSWV). In another particular embodiment, the plant pathogen is a virus, particularly the Tomato Spotted Wilt Virus (TSWV).

In another particular embodiment, the plant pathogen viroids are from a genus selected from the list consisting of Pospiviroid, Hostuviroid, Cocadviroid, Apscaviroid, Coleviroi, Avsunviroid, Pelamoviroid, Elaviroid, and combinations thereof. In a more particular embodiment, the plant pathogen viroids are selected from the list consisting of Potato spindle tuber viroid (PSTVd), Tomato chlorotic dwarf viroid (TCDVd), Mexican papita viroid; (MPVd), the Tomato planta macho viroid; (TPMVd), Chrysanthemum stunt viroid; (CSVd), Tomato apical stunt viroid; (TASVd), Iresine viroid 1 (IrVd-1), Columnea latent viroid; (CLVd), Hop stunt viroid, Coconut cadang-cadang viroid, Coconut tinangaja viroid (CTiVd), Hop latent viroid; (HLVd), Citrus viroid IV; (CVd-IV), Citrus viroid III (CVd-III), Apple dimple fruit viroid; (ADFVd), Grapevine yellow speckle 1 viroid (GVYSd-1), Grapevine yellow speckle viroid 2 (GVYSd-2), Citrus bent leaf viroid (CBLVd), Pear blister canker viroid; (PBCVd), Australian grapevine viroid (AGVd), Coleus blumei viroid 2 (CbVd-2), Coleus blumei viroid 3 (CbVd-3), Avocado sunblotch viroid, Peach latent mosaic viroid, Eggplant latent viroid and combinations thereof.

In a particular embodiment, the one or more plant pathogens are viroids. In another particular embodiment, the plant pathogens include the Potato spindle tuber viroid (PSTVd). In another particular embodiment, the plant pathogen is a viroid, particularly the PSTVd.

In another embodiment, the plant pathogen insects are selected from the insects disclosed in Barathi et al., (2023), supra, particularly from the list of insects disclosed in tables 2 and 3 of Barathi et al. (2023) *supra.* In another embodiment, the one or more plant pathogen insects are insects selected from the insects disclosed in Barathi et al., (2023), supra, particularly from the list of insects disclosed in tables 2 and 3 of Barathi et al. (2023) *supra.*

In another embodiment, the plant pathogen fungus are selected from the fungus disclosed in Barathi et al., (2023), supra, particularly from the list of fungus disclosed in table 4 of said document (Barathi et al. (2023) *supra).* In another embodiment, the one or more plant pathogen are fungus selected from the fungus disclosed in Barathi et al., (2023), supra, particularly from the list of fungus disclosed in table 4 of said document (Barathi et al. (2023) *supra).*

In another embodiment, the plant pathogen nematodes are selected from the nematodes disclosed in Barathi et al., (2023), supra, particularly from the list of nematodes indicated in table 6 of said document (Barathi et al. (2023) *supra).* In another emdboiment, the one or more plant pathogens are nematodes selected from the nematodes disclosed in Barathi et al., (2023), supra, particularly from the list of nematodes indicated in table 6 of said document (Barathi et al. (2023) *supra).*

In a particular embodiment of the first aspect, the host plant of the one or more plant pathogens are each one from a genus selected from the list consisting of pathogen is from a genus selected from the list consisting of *Solanum sp, Arabidopsis sp., Nicotiana sp., Pyrus sp, Capsicum sp., Citrullus sp., Cucumis sp., Cucurbita sp., Triticum sp., Oryza sp., Pisum sp., Cicersp., and Citrus sp.,* particularly are each one from a species selected from the list consisting of *Solanum Lycopersicum, Arabidopsis thaliana, Nicotiana benthamiana, Pyrus communis L., Solanum tuberosum, Solanum melongena, Capsicum annuum, Cucumis melo, Cucumis sativus, Citrullus lanatus, Cucurbita moschata, Zea mays, Triticum aestivum, Triticum turgidum, Oryza sativa, Pisum sativum, Cicer arietinum, Citrus sinensis, Citrus limon, Citrus paradisi and Citrus reticulata*.In another particular embodiment, the host plant of the one or more plant pathogens are each one selected from the plants disclosed in Barathi et al. (2023) *supra,* particularly in tables 1, 2, 4-7 of Barathi et al. (2023) *supra.* In a particular embodiment of the first aspect, the host plant of the plant pathogen is from a genus selected from the list consisting of pathogen is from a genus selected from the list consisting of *Solanum sp, Arabidopsis sp., Nicotiana sp., Pyrus sp, Capsicum sp., Citrullus sp., Cucumis sp., Cucurbita sp., Triticum sp., Oryza sp., Pisum sp., Cicersp., and Citrus sp.,* particularly is from a species selected from the list consisting of *Solanum lycopersicum, Arabidopsis thaliana, Nicotiana benthamiana, Pyrus communis L., Solanum tuberosum, Solanum melongena, Capsicum annuum, Cucumis melo, Cucumis sativus, Citrullus lanatus, Cucurbita moschata, Zea mays, Triticum aestivum, Triticum turgidum, Oryza sativa, Pisum sativum, Cicer arietinum, Citrus sinensis, Citrus limon, Citrus paradisi and Citrus reticulata*.In another particular embodiment, the host plant of the one or more plant pathogens are each one selected from the plants disclosed in Barathi et al. (2023) *supra,* particularly in tables 1, 2, 4-7 of Barathi et al. (2023) *supra.*

In a more particular embodiment, the host plant of the one or more plant pathogens are each one from a genus selected from the list consisting of pathogen is from a genus selected from the list consisting of *Solanum sp, Arabidopsis sp., Nicotiana sp., Pyrus sp.,* particularly are each one from a species selected from the list consisting of *Solanum lycopersicum, Arabidopsis thaliana, Nicotiana benthamiana, Pyrus communis L..* In another particular embodiment, the host plant of the one or more plant pathogens are each one from a genus selected from the list consisting of pathogen is from a genus selected from the list consisting of *Solanum sp, Arabidopsis sp., and Nicotiana sp.,* particularly are each one from a species selected from the list consisting of *Solanum lycopersicum, Arabidopsis thaliana, Nicotiana benthamiana.* In another particular embodiment, the host plant of the one or more plant pathogens are each one from a genus selected from the list consisting of pathogen is from the genus *Solanum sp.,* particularly from the species *Solanum Lycopersicum.*

In another particular embodiment of the first aspect, the host plant of the one or more plant pathogens are each one as the plant defined in the eighth aspect of the invention. In another particular embodiment of the first aspect, the host plant of the plant pathogen is as the plant defined in the eighth aspect of the invention.

In a particular embodiment, the one or more genes in the genome of one or more plant pathogens, are selected from those disclosed in Barathi et al. (2023) *supra* for the corresponding plant pathogen.

Thus, in a particular embodiment of the first aspect, the plant pathogen viruses and the one or more genes targeted by the siRNAs of region R3 are selected from table 5 of Barathi et al. (2023) *supra,* wherein the one or more genes are selected from the ones indicated therein for the corresponding plant pathogen. In another particular embodiment, the plant pathogen insects and the one or more genes targeted by the siRNAs of region R3 are selected from tables 2 and 3 of Barathi et al. (2023) *supra.,* wherein the one or more genes are selected from the ones indicated therein for the corresponding plant pathogen In another particular embodiment, the plant pathogen fungus and the one or more genes targeted by the siRNAs of region R3 are selected from table 4 of Barathi et al. (2023) *supra,* wherein the one or more genes are selected from the ones indicated therein for the corresponding plant pathogen. In another particular embodiment, the plant pathogen nematodes and the one or more genes targeted by the siRNAs of region R3 are selected from table 6 of Barathi et al. (2023) *supra,* wherein the one or more genes are selected from the ones indicated therein for the corresponding plant pathogen.

In a particular embodiment, the one or more plant pathogens are viruses, as defined herein, an the one or more genes targeted by the siRNA of region R3 are one or more genes encoding a viral protein selected from the list consisting of coat protein (CP), movement protein (MP), RNA polymerases, RNA-dependent RNA polymerase (RdRp), viral suppressors of RNA silencing (VSRs) and combinations thereof, particularly wherein the virus is the TSWV.

In a particular embodiment, the one or more genes targeted by the one or more siRNAs of region R3 are genes encoding a viral protein selected from the list consisting of RdRp or the putative movement protein (NSm), more particularly, wherein the plant pathogen is the virus TSWV.

Methods for determining the sequence of an siRNA that can be used to target a specific gene, as those just indicated, are well known by an expert in the field. Said methods include, for instance, the use of the P-SAMS script (P-SAMS script (https://github.com/carringtonlab/p-sams) as disclosed in Fahlgren,N , et al., (2016) P-SAMS: a web site for plant artificial microRNA and synthetic trans-acting small interfering RNA design. Bioinformatics, 32, 157-8. In addition, functional assays can be performed, using 'B/c' compatible vectors, as those mentioned in Fahlgren,N , et al., (2016) *supra ,* or described in Carbonell A. et al., (2019), Design and High-Throughput Generation of Artificial Small RNA Constructs for Plants. Methods Mol. Biol. 1932:247-260, or described in the examples below.

In an embodiment, the RNA sequences of the one or more siRNAs of region R3 are selected from the sequences provided in table 1 in Carbonell A. et al., (2019), Fast-forward identification of highly effective artificial small RNAs against different Tomato spotted wilt virus isolates. MPMI, 32, 142-156. In a more particular embodiment, the RNA sequences of the one or more siRNAs of region R3 comprise at least one sequence selected from the sequences provided in table 1 in Carbonell A. et al., (2019) supra. In a more particular embodiment, region R3 consists of the sequence of one siRNA selected from the sequences provided in table 1 in Carbonell A. et al., (2019) supra.

In a more particular embodiment, the RNA sequences of the one or more siRNAs of region R3 are selected from selected from the list consisting of SEQ ID NO. 11 (UGUAAGACGUGAUUGUGUCCU), SEQ ID NO. 12 (UAUCAGCUCUGGGUGAAUCGG), SEQ ID NO. 13 (UUGGUAUAGUGGGGCAUACCG), and SEQ ID NO. 14 (UCAGAGUGCACAAUCCAUCUU), particularly, wherein the plant pathogen is the TSWV. In another particular embodiment, the one or more siRNAs of region R3 comprise at least one sequence selected from the list consisting of SEQ ID NO. 11 (UGUAAGACGUGAUUGUGUCCU), SEQ ID NO. 12 (UAUCAGCUCUGGGUGAAUCGG), SEQ ID NO. 13 (UUGGUAUAGUGGGGCAUACCG), and SEQ ID NO. 14 (UCAGAGUGCACAAUCCAUCUU), particularly, wherein the plant pathogen is the TSWV. In another particular embodiment, the one or more siRNAs of region R3 comprise at least two siRNAs each one with a sequence selected from the list consisting of SEQ ID NO. 11 (UGUAAGACGUGAUUGUGUCCU), SEQ ID NO. 12 (UAUCAGCUCUGGGUGAAUCGG), SEQ ID NO. 13 (UUGGUAUAGUGGGGCAUACCG), and SEQ ID NO. 14 (UCAGAGUGCACAAUCCAUCUU), particularly, wherein the plant pathogen is the TSWV.. In another particular embodiment, the one or more siRNAs of region R3 comprise at least three siRNAs each one with a sequence selected from the list consisting of SEQ ID NO. 11 (UGUAAGACGUGAUUGUGUCCU), SEQ ID NO. 12 (UAUCAGCUCUGGGUGAAUCGG), SEQ ID NO. 13 (UUGGUAUAGUGGGGCAUACCG), and SEQ ID NO. 14 (UCAGAGUGCACAAUCCAUCUU), particularly, wherein the plant pathogen is the TSWV.. In another particular embodiment, the one or more siRNAs of region R3 comprise the siRNAs with sequence SEQ ID NO. 11 (UGUAAGACGUGAUUGUGUCCU), SEQ ID NO. 12 (UAUCAGCUCUGGGUGAAUCGG), SEQ ID NO. 13 (UUGGUAUAGUGGGGCAUACCG), and SEQ ID NO. 14 (UCAGAGUGCACAAUCCAUCUU), particularly, wherein the plant pathogen is the TSWV.. In another particular embodiment, the one or more siRNAs of region R3 are 4 siRNAs with sequence SEQ ID NO. 11 (UGUAAGACGUGAUUGUGUCCU), SEQ ID NO. 12 (UAUCAGCUCUGGGUGAAUCGG), SEQ ID NO. 13 (UUGGUAUAGUGGGGCAUACCG), and SEQ ID NO. 14 (UCAGAGUGCACAAUCCAUCUU), particularly, wherein the plant pathogen is the TSWV In another particular embodiment, region R3 consists of the sequence of one siRNA selected from the list consisting of SEQ ID NO. 11 (UGUAAGACGUGAUUGUGUCCU), SEQ ID NO. 12 (UAUCAGCUCUGGGUGAAUCGG), SEQ ID NO. 13 (UUGGUAUAGUGGGGCAUACCG), and SEQ ID NO. 14 (UCAGAGUGCACAAUCCAUCUU), particularly consists of SEQ ID NO. 11 (UGUAAGACGUGAUUGUGUCCU), more particularly, wherein the plant pathogen is the TSWV.

In another embodiment, the RNA sequence of the one or more siRNAs of region R3 are selected from selected from the list consisting of SEQ ID NO. 15 (UCGGCCGCUGGGCACUCCCCU), SEQ ID NO. 16 (UGCGGGCGCGAGGAAGGACAG), SEQ ID NO. 17 (UUUCCACCGGGUAGUAGCCGU), SEQ ID NO: 18 (UUAGUUCCGAGGAACCAACUC), , SEQ ID NO: 19 (UCAAGGGCUAAACACCCUCGG), SEQ ID NO. 20 (UGGAACCGCAGUUGGUUCCUG), SEQ ID NO. 21 (UGAAGCUCCCGAGAACCGCUA), SEQ ID NO: 22 (UGUCGCUUCGGCUACUACCCC), SEQ ID NO: 23 (UGUCCUUCCUCGCGCCCGCAC), SEQ ID NO. 24 (UGGUUCACACCUGACCUCCUC), SEQ ID NO. 25 (UCCCGGGGAAACCUGGAGCGU), particularly, wherein the plant pathogen is the PSTVd. In a more particular embodiment, the RNA sequence of the one or more siRNAS of region R3 are selected from the list consisting of SEQ ID NO. 16, SEQ ID NO. 18, SEQ ID NO. 23, and SEQ ID NO. 25, particularly wherein the plant pathogen is the PsTVd. In another particular embodiment, the one or more siRNAs of region R3 comprise at least two siRNAs each one with a sequence selected from the list consisting of SEQ ID NO. 15, SEQ ID NO. 16, SEQ ID NO. 17, SEQ ID NO: 18, SEQ ID NO: 19, SEQ ID NO. 20, SEQ ID NO. 21, SEQ ID NO: 22, SEQ ID NO: 23, SEQ ID NO. 24, SEQ ID NO. 25, particularly from the list consisting of SEQ ID NO. 16, SEQ ID NO. 18, SEQ ID NO. 23, and SEQ ID NO. 25, more particularly wherein the plant pathogen is the PsTVd. In another particular embodiment, the one or more siRNAs of region R3 comprise at least 3 siRNAs each one with a sequence selected from the list consisting of SEQ ID NO. 15, SEQ ID NO. 16, SEQ ID NO. 17, SEQ ID NO: 18, SEQ ID NO: 19, SEQ ID NO. 20, SEQ ID NO. 21, SEQ ID NO: 22, SEQ ID NO: 23, SEQ ID NO. 24, SEQ ID NO. 25, particularly from the list consisting of SEQ ID NO. 16, SEQ ID NO. 18, SEQ ID NO. 23, and SEQ ID NO. 25, more particularly wherein the plant pathogen is the PsTVd. In another particular embodiment, the one or more siRNAs of region R3 comprise at least 4 siRNAs each one with a sequence selected from the list consisting of SEQ ID NO. 15, SEQ ID NO. 16, SEQ ID NO. 17, SEQ ID NO: 18, SEQ ID NO: 19, SEQ ID NO. 20, SEQ ID NO. 21, SEQ ID NO: 22, SEQ ID NO: 23, SEQ ID NO. 24, SEQ ID NO. 25, particularly from the list consisting of SEQ ID NO. 16, SEQ ID NO. 18, SEQ ID NO. 23, and SEQ ID NO. 25, more particularly wherein the plant pathogen is the PsTVd. In another particular embodiment, the one or more siRNAs of region R3 are 4 siRNAs each one with a sequence selected from the list consisting of SEQ ID NO. 15, SEQ ID NO. 16, SEQ ID NO. 17, SEQ ID NO: 18, SEQ ID NO: 19, SEQ ID NO. 20, SEQ ID NO. 21, SEQ ID NO: 22, SEQ ID NO: 23, SEQ ID NO. 24, SEQ ID NO. 25, particularly from the list consisting of SEQ ID NO. 16, SEQ ID NO. 18, SEQ ID NO. 23, and SEQ ID NO. 25, more particularly wherein the plant pathogen is the PsTVd .

In a particular embodiment, the one or more siRNAs of region R3 target one or more genes in the genome of a plant. In a particular embodiment, region R3 consists of the sequence of one siRNA that targets one gene in the genome of a plant. In another particular embodiment, region R3 consists of the RNA sequence of at least 2, at least 3, at least 4, at least 5, at least 6, at least 7, at least 8, at least 9, or at least 10 siRNAs, particularly of at least 4 siRNAs, wherein the siRNA sequences of region R3 are directly linked to each other, and the siRNAs of region R3 target one or more genes in the genome of a plant. In another particular embodiment, region R3 consists of the RNA sequence of at least 2, at least 3, at least 4, at least 5, at least 6, at least 7, at least 8, at least 9, or at least 10 siRNAs, particularly of at least 4 siRNAs, wherein the siRNA sequences are directly linked to each other, and the at least 2, at least 3, at least 4, at least 5, at least 6, at least 7, at least 8, at least 9, or at least 10 siRNAs, particularly the at least 4 siRNAs target one or more genes in the genome of a plant. In another particular embodiment, region R3 consists of the RNA sequence of at least 2, at least 3, at least 4, at least 5, at least 6, at least 7, at least 8, at least 9, or at least 10 siRNAs, particularly of at least 4 siRNAs, wherein the siRNA sequences of the at least 2, at least 3, at least 4, at least 5, at least 6, at least 7, at least 8, at least 9, or at least 10 siRNAs, particularly of the at least 4 siRNAs are directly linked to each other and correspond to sequences of siRNAs that target one or more genes in the genome of a plant.

In another particular embodiment, region R3 consists of the RNA sequence of at least 2, at least 3, at least 4, at least 5, at least 6, at least 7, at least 8, at least 9, or at least 10 siRNAs, particularly of at least 4 siRNAs, wherein the siRNA sequences of region R3 are directly linked to each other, and the siRNAs of region R3 target one gene in the genome of a plant.

In another particular embodiment, region R3 consists of the RNA sequence of at least 2, at least 3, at least 4, at least 5, at least 6, at least 7, at least 8, at least 9, or at least 10 siRNAs, particularly of at least 4 siRNAs, wherein the siRNA sequences of region R3 are directly linked to each other, and the siRNAs of region R3 target at least 2 genes in the genome of a plant.

In a particular embodiment, region R3 consists of the RNA sequence of at least 2, at least 3, at least 4, at least 5, at least 6, at least 7, at least 8, at least 9, or at least 10 siRNAs, particularly of at least 4 siRNAs, wherein the siRNA sequences of region R3 are directly linked to each other, and the siRNAs of region R3 target at least one gene in the genome of one or more plant pathogens and at least one gene in the genome of a plant. In another particular embodiment, region R3 consists of the RNA sequence of at least 2, at least 3, at least 4, at least 5, at least 6, at least 7, at least 8, at least 9, or at least 10 siRNAs, particularly of at least 4 siRNAs, wherein the siRNA sequences of region R3 are directly linked to each other, and the siRNAs of region R3 target at least one gene in the genome of a plant pathogens and at least one gene in the genome of a plant.

In an another embodiment, the one or more genes in the genome of a plant targeted by the one or more siRNAs of region R3 are one or more genes encoding a protein selected from the list consisting of SFT (single flower truss), particularly from *Solanum lycopersium,* TFL1 (terminal flower 1), particularly from *Pyrus communis L.,* DET1 (DE-ETIOLATED 1), particularly from *Solanum lycopersicum,* ALS (acetolactate synthase), ACCase (acetyl-CoA carboxylase) and EPSPS (5-enolpyruvylshikimate-3-phosphate). In a particular embodiment, the one or more genes in the genome of a plant targeted by the one or more siRNAs of region R3 are selected from those disclosed in Barathi et al. 2023, *supra,* particularly in tables 1 and 7 of Barathi et al. 2023, *supra.*

In a particular embodiment, the plant comprising the gene targeted by the one or more siRNAs of region R3 is a genus selected from the list consisting of *Solanum sp, Arabidopsis sp., Nicotiana sp., Pyrus sp, Capsicum sp., Citrullus sp., Cucumis sp., Cucurbita sp., Triticum sp., Oryza sp., Pisum sp., Cicer sp., and Citrus sp.,* particularly is from a species selected from the list consisting of *Solanum lycopersicum, Arabidopsis thaliana, Nicotiana benthamiana, Pyrus communis L., Solanum tuberosum, Solanum melongena, Capsicum annuum, Cucumis melo, Cucumis sativus, Citrullus lanatus, Cucurbita moschata, Zea mays, Triticum aestivum, Triticum turgidum, Oryza sativa, Pisum sativum, Cicer arietinum, Citrus sinensis, Citrus limon, Citrus paradisi and Citrus reticulata....* In another particular embodiment, the plant comprising the gene targeted by the one or more siRNAs of region R3 is selected from the plants disclosed in Barathi et al. (2023) *supra,* particularly in tables 1, 2, 4-7 of Barathi et al. (2023) *supra.* In another particular embodiment of the first aspect, plant comprising the gene targeted by the one or more siRNAs of region R3 is the plant as defined in the eighth aspect of the invention.,

In a particular embodiment, the one or more siRNAs of the invention are synthetic, i.e. are not found in nature. Methods to design an siRNA against a particular gene have been provided above. In another particular embodiment, the one or more siRNAs of region R3 are found in nature, more particularly, are endogenously expressed in the plant that is the host of the plant pathogen comprising the one or more siRNA target genes, as any of the plants listed above, or in the plant that comprise the one or more siRNA target genes, as any of the plants listed above. In another particular embodiment, the one or more siRNAs of region R3 are found in nature, more particularly, are endogenously expressed in the plant of the invention. In a particular embodiment, the host plant of the plant pathogens and/or the plant that comprise the one or more siRNA target genes correspond to the plant of the invention as defined below.

In a second aspect, the invention provides a viral vector comprising the reverse complement sequence of the sequence of the viral vector as defined in the first aspect of the invention. In a particular embodiment, the viral vector of the second aspect of the invention comprises a heterologous sequence encoding or comprising the reverse complement of the RNA sequence S1 defined in the first aspect of the invention. In another particular embodiment, the terms, embodiments and definitions of the first aspect of the invention apply to the second aspect of the invention, wherein the RNA sequence S1, regions R1, R2, and R3, defined in any of said embodiments and definitions, is substituted by the corresponding reverse complement sequence.

In a particular embodiment, the one or more siRNAs of region R3 are expressed from the viral vector of the first or second aspect of the invention, particularly in a plant cell comprising said viral vector. In a particular embodiment, the plant cell is a cell from the plant of the invention as defined below.

As well understood by a skilled person, a first RNA sequence upstream a second RNA sequence or RNA region, as described in any aspects of the invention, indicates that the 3' end nucleotide of the first RNA sequence is directly linked to the 5'-end nucleotide of the second RNA sequence of said RNA region. Thus, a first RNA sequence upstream a second RNA sequence or RNA region, as described in any aspects of the invention, is herein indicated in a 5'- to 3'-end direction. Similarly, a first RNA sequence downstream a second RNA sequence or RNA region, as described in any aspects of the invention, indicates that the 5' end nucleotide of the first RNA sequence is directly linked to the 3'-end nucleotide of the second RNA sequence of said RNA region. Concomitantly, a first RNA sequence downstream a second RNA sequence or RNA region, as described in any aspects of the invention, is herein also indicated in a 5'- to 3'- end direction.

In a particular embodiment of the first aspect, the sequence of regions R1 and R2 directly linked to each other (wherein the 3' end of R1 is directly linked to the 5' end of region R2) consists of SEQ ID NO. 77, SEQ ID NO. 78, or SEQ ID NO. 79.

### II-The virus of the invention

In a third aspect, the invention provides a virus encoded by or comprising the viral vector of the first or the second aspect of the invention. In a particular embodiment, the virus of the third aspect of the invention induces mild symptoms or is asymptomatic in the plant of the invention, as defined below. Thus, infection of the plant of the invention with the virus of the invention causes mild or no symptoms in the plant. In a particular embodiment, the virus of the invention is an attenuated virus. In another particular embodiment, the viral vector of the invention is modified to express an attenuated virus in the plant comprising the viral vector, wherein the plant is as just defined.

The term "attenuated virus", as used herein, refers to a virus that produces milder damage in the infected organism than the wild-type counterpart from which it is derived.

In a particular embodiment, the virus of the invention is variant of the PVX, TRV, TMV, TGMV, CMV, BSMV, BMV, FoMV,BYDV, WDV, or CabLCV, particularly of the PVX or TRV. In a particular embodiment, the virus of the invention derives from PVX, TRV, TMV, TGMV, CMV, BSMV, BMV, FoMV,BYDV, WDV, or CabLCV, particularly from the PVX or TRV.

In a particular embodiment, the virus of the invention expresses the RNA sequence S1 of the invention in a host plant of said virus, particularly in the plant of the invention. In another particular embodiment, the virus of the invention expresses the one or more siRNAs of region R3 in the plant of the invention.

In another particular embodiment, the virus of the invention is a PVX, TRV, BYDV, WDV, or CabLCV, particularly a TRV or a PVX virus, more particularly a PVX virus, that expresses the RNA sequence S1 of the invention in a host plant of said virus, particularly in the plant of the invention. In another particular embodiment, the virus of the invention is a PVX, TRV, BYDV, WDV, or CaLCuV, particularly a TRV or a PVX virus, more particularly a PVX virus that expresses the one or more siRNAs of region R3 in the plant of the invention.

### III- The polynucleotides of the invention

In a fourth aspect, the invention provides an RNA polynucleotide comprising the RNA sequence S1 as defined in the first aspect of the invention, provided that the RNA polynucleotide does not comprise:
(i) in the region upstream of region R1, in a 5'- to 3'- end direction, the sequence immediately upstream the miR173 target site in the Trans-acting siRNA1C (TAS1C) primary precursor, and/or
(ii) in the region downstream of region R3, in a 5'- to 3'- end direction, the sequence immediately downstream sequence SEQ ID NO. 26 in the TAS1C primary precursor.

In a fifth aspect, the invention provides a DNA polynucleotide encoding the polynucleotide as defined in the fourth aspect of the invention.

In a particular embodiment, the RNA polynucleotide of the fourth aspect of the invention comprises the RNA sequence S1 as defined in the first aspect, provided that the RNA polynucleotide does not comprise in the region upstream of region R1 a sequence of at least 10, at least 20, at least 30, at least 40, at least 50, at least 70, at least 100, at least 150, at least 180, at least 200, at least 210, at least 220, at least 230, or at least 232 nucleotides, particularly at least 100 nucleotides comprised in the region of immediately upstream the miR173 target site in the TAS1C primary precursor.

In a particular embodiment, the RNA polynucleotide of the fourth aspect of the invention comprises the RNA sequence S1 as defined in the first aspect, provided that the RNA polynucleotide does not comprise in the region upstream of region R1, the at least 10, at least 20, at least 30, at least 40, at least 50, at least 70, at least 100, at least 150, at least 180, at least 200, at least 210, at least 220, at least 230, or at least 232 nucleotides, particularly the at least 100 nucleotides immediately upstream the miR173 target site in the Trans-acting siRNA1C (TAS1C) primary precursor. In a particular embodiment, RNA polynucleotide of the fourth aspect of the invention comprises the RNA sequence S1 as defined in the first aspect, provided that the RNA polynucleotide does not comprise in the region upstream of region R1 SEQ ID NO. 27. In a particular embodiment, RNA polynucleotide of the fourth aspect of the invention comprises the RNA sequence S1 as defined in the first aspect, provided that the RNA polynucleotide does not comprise in the region upstream of region R1 SEQ ID NO. 29. In a particular embodiment, the region immediately upstream the miR173 target site in the TAS1C primary precursor consists of SEQ ID NO. 29.

In another particular embodiment, the RNA polynucleotide of the fourth aspect of the invention comprises the RNA sequence S1 as defined in the first aspect, provided that the RNA polynucleotide does not comprise in the region downstream of region R3, a sequence of at least 10, at least 20, at least 30, at least 40, at least 50, at least 70, at least 100, at least 150, at least 180, at least 200, at least 210, at least 220, at least 230, or at least 232 nucleotides, particularly at least 100 nucleotides comprised in the region immediately downstream sequence SEQ ID NO. 26 in the TAS1C primary precursor.

In a particular embodiment, the RNA polynucleotide of the fourth aspect of the invention comprises the RNA sequence S1 as defined in the first aspect, provided that the RNA polynucleotide does not comprise in the region downstream of region R3, the at least 10, at least 20, at least 30, at least 40, at least 50, at least 70, at least 100, at least 150, at least 180, at least 200, at least 210, at least 220, at least 230, or at least 232 nucleotides, particularly the at least 100 nucleotides immediately downstream sequence SEQ ID NO. 26 in the TAS1C primary precursor. In a particular embodiment, RNA polynucleotide of the fourth aspect of the invention comprises the RNA sequence S1 as defined in the first aspect, provided that the RNA polynucleotide does not comprise in the region downstream of region R3 SEQ ID NO. 28. In a particular embodiment, RNA polynucleotide of the fourth aspect of the invention comprises the RNA sequence S1 as defined in the first aspect, provided that the RNA polynucleotide does not comprise in the region downstream of region R3 SEQ ID NO. 30. In a particular embodiment, the region region immediately downstream sequence SEQ ID NO. 26 in the TAS1C primary precursor consists of SEQ ID NO. 30.

In another particular embodiment, the RNA polynucleotide of the fourth aspect of the invention comprises the RNA sequence S1 as defined in the first aspect, provided that the RNA polynucleotide does not comprise:
(i) in the region upstream of region R1, in a 5'- to 3'- end direction, a sequence comprised in the region of immediately upstream the miR173 target site in the TAS1C primary precursor of at least 150 nucleotides, and/or
(ii) in the region downstream of region R5, in a 5'- to 3'- end direction, a sequence comprised in the region immediately downstream sequence SEQ ID NO. 26 in the TAS1C primary precursor of at least 110 nucleotides.

In another particular embodiment, the RNA polynucleotide of the fourth aspect of the invention comprises the RNA sequence S1 as defined in the first aspect, provided that the RNA polynucleotide does not comprise:
(i) in the region upstream of region R1, in a 5'- to 3'- end direction, SEQ ID NO. 27, and/or
(ii) in the region downstream of region R5, in a 5'- to 3'- end direction, SEQ ID NO. 28.

In another particular embodiment, the RNA polynucleotide of the fourth aspect of the invention comprises the RNA sequence S1 as defined in the first aspect, provided that the RNA polynucleotide does not comprise:
(i) in the region upstream of region R1, in a 5'- to 3'- end direction, SEQ ID NO. 29, and/or
(ii) in the region downstream of region R5, in a 5'- to 3'- end direction, SEQ ID NO. 30.

In a particular embodiment, the length of the polynucleotide of the fourth aspect of the invention is of at least 50, at least 51, at least 52, at least 53, at least 54, at least at least 55, at least 70, at least 71, at least 72, at least 73, at least 74, at least 113, at least 112, at least 114, at least 115, at least 116, at least 117, at least 134, at least 136, at least 137, at least 138, at least 155, at least 156, at least 157, at least 158, at least 159, at least 178, at least 178, at least 179, at least 180, at least 200, at least 300, at least 400, at least 450, at least 451, at least 472, at least 493, at least 514, at least 600, at least 700, at least 800, at least 878, at least 899, at least 920, or at least 941 nucleotides, particularly at least 54 nucleotides, more particularly at least 117 nucleotides. In another particular embodiment, the length of the polynucleotide of the fourth aspect of the invention is of 50, 51, 52, 53, 54, 55, 70, 71, 72, 73, 74, 113, 112, 114, 115, 116, 117, 134, 135, 136, 137, 138, 155, 156, 157, 158, 159, 178, 178, 179, 180, 451, 472, 493, 514, 600, 878, 899, 920, or 941 nucleotides, particularly 878 nucleotides, more particularly 941 nucleotides. In another particular embodiment, the polynucleotide of the fourth aspect of the invention is of 451 nucleotides, more particularly of 514 nucleotides.

In a particular embodiment, the polynucleotide of the fourth aspect of the invention comprises a poly-A tail at the 3'end. In another particular embodiment, the polynucleotide of the fourth aspect of the invention comprises a cap structure at the 5' end. In a more particular embodiment, the polynucleotide of the fourth aspect of the invention comprises a poly-A tail at the 3'end and a cap structure at the 5' end.

In a particular embodiment, the sequence of the polynucleotide of the fourth aspect of the invention consists of the RNA sequence S1 and at least 1, at least 10, at least 20, at least 0, at least 100, at least 150, at least 250, at least 300, at least 350, at least 375, at least 380, at least 390, at least 395, at least 396 at least 397, at least 398, at least 399, at least 400, at least 450, at least 500, at least 600, at least 700, at least 710, at least 720, at least 730,n at least 735, at least 740, at least 750, at least 800, particularly at least 397 nucleotides upstream the RNA sequence S1. In another particular embodiment, the sequence of the polynucleotide of the fourth aspect of the invention consists of the RNA sequence S1 and at least 1, at least 10, at least 20, at least 0, at least 100, at least 150, atleast 250, at least 300, at least 350, at least 375, at least 380, at least 390, at least 395, at least 396 at least 397, at least 398, at least 399, at least 400, at least 450, at least 500, at least 600, at least 700, at least 710, at least 720, at least 730, at least 735, at least 740, at least 750, at least 800, particularly at least 735 nucleotides downstream the RNA sequence S1. In another particular embodiment, In another particular embodiment, the polynucleotide of the fourth aspect of the invention consists of the RNA sequence S1 and at least 1, particularly at least 397 nucleotides upstream the RNA sequence S1, and/or at least 1, at least 735 nucleotides downstream the RNA sequence S1.
In a particular embodiment, the sequence of the polynucleotide of the fourth aspect of the invention consists of the RNA sequence S1. In another particular embodiment, the sequence of the polynucleotide of the fourth aspect of the invention consists of the RNA sequence S1 and a poly-A tail at the 3'end. In another particular embodiment, the sequence of the polynucleotide of the fourth aspect of the invention consists of the RNA sequence S1 with a cap structure at the 5'end. In another particular embodiment, the sequence of the polynucleotide of the fourth aspect of the invention consists of the RNA sequence S1 with a cap structure at the 5' end and a poly-A tail at the 3'end. In another particular embodiment, the sequence of the polynucleotide of the fourth aspect of the invention consists of regions R1, R2 and R3 directly linked to each other as indicated in formula (i) (herein referred to as region F). In another particular embodiment, the sequence of the polynucleotide of the fourth aspect of the invention consists of the RNA sequence of region F, and a poly-A tail at the 3'end. In another particular embodiment, the sequence of the polynucleotide of the fourth aspect of the invention consists of the RNA sequence of region F with a cap structure at the 5'end. In another particular embodiment, the sequence of the polynucleotide of the fourth aspect of the invention consists of the RNA sequence of region F with a cap structure at the 5' end and a poly-A tail at the 3'end.

The term "poly-A tail" as used herein, refers to a long chain of adenine ribonucleotides that is added to the 3'end of an RNA molecule during RNA processing and is generally of 50-250 nucleotides long. The poly-A tail is known to increase the stability of the RNA molecule and to be involved in the translation of the mRNA molecules. In a particular embodiment, the poly-A tail of the polynucleotide of the fourth aspect of the invention is of 50, 70, 80, 90, 100, 120, 150, 170, 200, 220, or 250 adenine ribonucleotides.

The term "cap structure" as used herein, refers to a chemical modification of RNA molecules in eukaryotes, which increases the stability of the RNA and that is important for the transport of the RNA from the nucleus to the cytoplasm and for the subsequent translation of mRNAs by the ribosomes. In eukaryotes, the 5' cap consists of a guanine nucleotide connected to the RNA via an unusual 5' to 5' triphosphate linkage. This guanosine is methylated on the 7 position directly after capping in vivo by a methyltransferase. It is referred to as a 7-methylguanylate cap, abbreviated m7G. In a particular embodiment, the cap structure of the polynucleotide of the fourth aspect is an m7G cap.

### IV- The plasmid and bacterium of the invention

In a sixth aspect, the invention provides a plasmid comprising a sequence that encodes or corresponds to the sequence of the viral vector as defined in the first or second aspect of the invention, or the sequence encoding the polynucleotide as defined in the fourth aspect of the invention.

In a seventh aspect, the invention provides a bacterium that comprises the plasmid as defined in the sixth aspect of the invention.

In a particular embodiment, the plasmid of the sixth aspect is suitable for transfection in plant cells. In a more particular embodiment, the plasmid of the sixth aspect is suitable for transfection in plants mediated by the *Agrobacterium tumefaciens.* In other words, the plasmid of the sixth aspect of the invention is suitable for agroinfiltration. As well understood by a skilled person, plasmid suitable for plant transfection or agroinfiltration need to comprise a plasmids backbone suitable for those purposes. Plasmids backbones suitable for plant transfection and agroinfiltration are well known by an expert in the field. Non-limitative examples include those disclosed in the examples bellow. In a particular, the plasmid backbone of the plasmid of the sixth aspect of the invention is selected from the list consisting of pBIN, pGA, SEV, pEND4K, pBI, pCIB10, pMRK63, pGPTV, pCGN1547, pART, pGKB5, pMJD80 and 81, pPZP, pBINPLUS, pRT100, BIBAC, pCB, pGreen, pPZP-RCS2, pMDC, pRCS2, pEarleyGate, pGWTAC, pORE, pSITE, pMSP, pCAMBIA, PGD, *pMDC32, pLX-B2,* particularly, consists of the *pLX-B2* plasmid.. Thus, in a particular embodiment, the plasmid of the sixth aspect of the invention is aPMDC32 plasmid, or a pLX-B2 plasmid, particularly a pLX-B2 plasmid.

In another particular embodiment, the plasmid of the sixth aspect of the invention is a pENTR plasmid.
In a particular embodiment of the sixth aspect, the sequence that encodes or corresponds to the sequence of the viral vector as defined in the first or second aspect of the invention, or the sequence encoding the polynucleotide as defined in the fourth aspect of the invention, is operatively linked to an expression promoter in the plasmid of the sixth aspect of the invention. The term "operatively linked" has already been defined in the first aspect of the invention and also applies to the sixth aspect of the invention. Suitable expression promoters are known by an expert in the field. In a particular embodiment, the expression promoter is the 35S promoter from the plant pathogen Cauliflower mosaic virus (CaMV).

In a particular embodiment of the seventh aspect of the invention, the bacterium is from the genus *Agrobacterium,* particularly from the species *Agrobacterium tumefacines.*

In a particular embodiment, the embodiments and definitions of the first, second, third, fourth and fifth aspects of the invention apply to the sixth and seventh aspects of the invention.

### V- Plants and plant extracts of the invention and methods for obtaining them

In an eighth aspect, the invention provides a plant that comprises the viral vector as defined in the first or second aspect of the invention, the virus as defined in the third aspect of the invention, the polynucleotide as defined in the fourth or fifth aspects of the invention, the plasmid as defined in the sixth aspect and/or the bacterium as defined in the seventh aspect.

In a particular embodiment, the plant of the eighth aspect of the invention comprises the viral vector as defined in the first or second aspect of the invention, the virus as defined in the third aspect of the invention, the polynucleotide as defined in the fourth or fifth aspect, the plasmid as defined in the sixth aspect and/or the bacterium as defined in the seventh aspect, in the cells of the plant, particularly in the cells from at least one organ of the plant. In a particular embodiment, the at least one organ of the plant is selected from the list consisting of leaves, the stem, roots, flowers, fruits and combinations thereof. In a particular embodiment, cells of the plant that comprise the viral vector as defined in the first or second aspect of the invention, the virus as defined in the third aspect of the invention, the polynucleotide as defined in the fourth or fifth aspect, the plasmid as defined in the sixth aspect and/or the bacterium as defined in the seventh aspect, are the cells of at least one leaf, more particularly of one leaf of the plant.

In another embodiment, the plant of the eighth aspect of the invention comprises in cells of all the organs of the plant, the viral vector as defined in the first or second aspect of the invention, the virus as defined in the third aspect of the invention, the polynucleotide as defined in the fourth or fifth aspect, the plasmid as defined in the sixth aspect and/or the bacterium as defined in the seventh aspect. In another particular embodiment, plant of the eighth aspect of the invention, comprises in all the cells of the plant, the viral vector as defined in the first or second aspect of the invention, the virus as defined in the third aspect of the invention, the polynucleotide as defined in the fourth or fifth aspect, the plasmid as defined in the sixth aspect and/or the bacterium as defined in the seventh aspect.

In a particular embodiment, the fact that the plant, particularly the cells of the plant as described in the paragraph above, comprise the virus as defined in the third aspect of the invention, refers to the fact that the plant, particularly said cells of the plant, are infected with the virus as defined in the third aspect of the invention. The fact that the plant, particularly the cells of the plant as described in the paragraph above, comprise the bacterium as defined in the seventh aspect of the invention, refers to the fact that the plant, particularly said cells of the plant, are infected with the bacterium as defined in the seventh aspect of the invention.

In a particular embodiment, the plant of the eighth aspect of the invention comprises the virus of the invention, particularly in cells of the plant of the invention, wherein the virus of the invention replicates within the cells of the plant, particularly, wherein the virus of the invention replicates within the cells of the plant and spreads between cells of the plant, particularly between adjacent cells of the plant and/or by the vascular system of the plant. In a particular embodiment, the plant of the invention presents the symptoms of infection by the virus from which the viral vector of the invention derives. In another particular embodiment, the plant of the invention does not present symptoms of infection by the virus from which the viral vector of the invention derives.

In a particular embodiment, the plant of the eighth aspect is resistant to the one or more plant pathogens as defined in any of the embodiments of the first aspect of the invention. In another embodiment, the plant of the eighth aspect is tolerant to the one or more plant pathogens as defined in any of the embodiments of the first aspect of the invention. In a particular embodiment, the plant of the invention comprises the one or more plant pathogens as defined in the first aspect of the invention.

The term "resistant to the one or more plant pathogen", as used herein, refers to a plant that inhibits the infection, multiplication and/or invasion of the said one or more plant pathogen within the plant. Methods to determine if a plant is resistant to a pathogen are well known by an expert in the field and include determining the plant pathogen concentration in an organ of the plant exposed to the plant pathogen, as compared to the concentration in the same organ in a control plant not resistant to the plant pathogen and equivalently exposed to the plant pathogen. If the concentration of plant pathogen in the organ of the plant analyzed is lower than in the organ of the control plant, it is considered that the plant is resistant to the plant pathogen. In a particular embodiment, the plant is considered to be resistant to the plant pathogen if the concentration of the plant pathogen determined in the organ of the plant analyzed is at least 20%, at least 30%, at least 40%, at least 50%m, at least 60% at least 70%, at least 80%, at least 90%, at least 95%, at least 98% lower than in the organ of the control plant. Methods to determine the concentration of a plant pathogen in a plant organ are well known by an expert in the field. For instance, it can be determined by the quantitative PCR (qPCR), Quantitative reverse transcription PCR (RT-qPCR), or flow cytometry. In a particular embodiment, the control plant not resistant to the plant pathogen is a plant of the same species, the same age and grown under the same conditions as the plant being analyzed, but that does not comprise the viral vector as defined in the first or second aspect of the invention, the virus as defined in the third aspect of the invention, the polynucleotide as defined in the fourth or fifth aspect, the plasmid as defined in the sixth aspect nor the bacterium as defined in the seventh aspect.

The term "tolerant to one or more a plant pathogen", as used herein, refers to a plant that the one or more pathogens can infect, replicate in it, and invade without leading to severe symptoms or diminishing plant growth or marketable yield. Methods to determine if a plant is tolerant to a plant pathogen include determining: a) the level of symptoms associated to said pathogen developed by a plant exposed to the plant pathogen, and comparing them to the level of symptoms developed by a control plant equivalently exposed to the plant pathogen; and b) the concentration of plant pathogen in an organ of the plant analyzed exposed to said plant pathogen, as indicated in the definition of "resistant plant". If the level of symptoms developed by the plant analyzed is lower than those developed by the control plant, and the concentration of plant pathogen in the plant analyzed is comparable to that in the control plant, it is considered that the plant is tolerant to the plant pathogen. In a particular embodiment, the plant is considered to be tolerant to the plant pathogen if the level of symptoms associated to said pathogen in the plant analyzed is at least 20%, at least 30%, at least 40%, at least 50%m, at least 60% at least 70%, at least 80%, at least 90%, at least 95%, at least 98%, particularly at least 60% lower than in the control plant, and the concentration of the plant pathogen determined in the organ of the plant analyzed is 75%-125%, 80%-120%, 90-110% of the concentration in the organ of the control plant. Methods to measure the level of symptoms in a plant are well known by an expert in the field and include, for instance, those disclosed in the examples below. Methods to determine the concentration of a plant pathogen are provided in the definition of "resistant to the plant pathogen".

In a particular embodiment, the one or more plant pathogens mentioned in any of the embodiments of the eighth aspect of the invention are selected from the plant pathogens disclosed in the first aspect of the invention. In a particular embodiment, the one or more plant pathogen mentioned in the eighth aspect of the invention are viruses, particularly selected from the list consisting of the TSWV, ToBRFV, ToLCNDV, and PsTVd, particularly consist of the TSWV.

In another embodiment, the plant of the eighth aspect of the invention shows at least one improved trait, particularly as compared to a control plant. In a particular embodiment, the at least one improved trait, particularly as compared to the control plant, is selected from the list consisting of crop yield, shelf life of fruits, shelf life of vegetables, nutritional value, organoleptic properties, abiotic stress resistance (for instance, to drought, salinity, high temperatures, or cold), or male sterility. In a particular embodiment, the at least one improved trait, particularly as compared to the control plant, is selected from those disclosed in tables 1 and in table 7 of Barathi et al., (2023), *supra.*

In a particular embodiment, the plant of the eighth aspect of the invention shows at least one improved trait, particularly as compared to a control plant wherein said at least one trait is at least 10%, 20%, 30%, 40%, 50%, 75%, 90%, 95%, 100%, 150%, 200%, 250%, 300%, 350%, 400% higher, particularly at least 20% higher, in the plant of the eighth aspect of the invention than in a control plant. In a more particular embodiment, the at least one improved trait is at least 50% higher in the plant of the eighth aspect of the invention than in a control plant. In a yet more particular embodiment, the at least one improved trait is at least 100% higher in the plant of the eighth aspect of the invention than in a control plant. Methods to compare the level of plant traits between different plants, as those disclosed above, are well known by an expert in the field, and include analyzing the plant characters and protein levels mentioned in Barathi et al. (2023) *supra* for each trait.

In a particular embodiment, the control plant indicated in the embodiments of the plant of the eighth with at least one improved trait, particularly in the two previous paragraphs, is a plant of the same species, the same age and grown in the same conditions as the plant of the eighth aspect of the invention, but that does not comprise the viral vector as defined in the first or second aspect of the invention, the virus as defined in the third aspect of the invention, the polynucleotide as defined in the fourth or fifth aspect, the plasmid as defined in the sixth aspect nor the bacterium as defined in the seventh aspect.

In an embodiment, the plant of the fourth aspect is a non-transgenic plant. The term "non-transgenic plant", as used herein, refers to the term commonly known by an expert in the field. Particularly, it refers to a plant that has not been genetically modified with genetic engineering techniques. Thus, the genome of the non-transgenic plant only comprises nucleic acid sequences that can be part of the genome of the plant upon mating and/or natural recombination. In a particular embodiment of the eighth aspect, the non-transgenic plant does not comprise artificial sequences integrated in its genome, wherein the artificial sequences is any sequence not found in nature in the genome of said plant. In a particular embodiment of the eighth aspect, the non-transgenic plant does not comprise heterologous sequences integrated in its genome, wherein the heterologous sequences is as defined in the first aspect of the invention. In a particular embodiment of the eighth aspect of the invention, nor the sequence of the viral vector of the first or second aspect of the invention, nor the sequence of the polynucleotide of the fifth aspect of the invention, nor the sequence of the plasmid of the sixth aspect of the invention is comprised in the genome of the plant as defined in the eighth aspect of the invention. In another particular embodiment, the plant of the eighth aspect of the invention comprises heterologous sequences, or is a transgenic plant, but does not comprise the sequence of the viral vector of the first or second aspect of the invention, nor the sequence of the polynucleotide of the fifth aspect of the invention, nor the sequence of the plasmid of the sixth aspect of the invention. In another particular embodiment, the plant of the eighth aspect of the invention does not comprise heterologous or artificial sequences integrated in its genome, nor the sequence of the viral vector of the first or second aspect of the invention, nor the sequence of the polynucleotide of the fifth aspect of the invention, nor the sequence of the plasmid of the sixth aspect of the invention.

In a particular embodiment of the eighth aspect, the plant of the invention is non-transgenic and comprises the virus as defined in the third aspect of the invention. In a more particular emobdiment, plant of the invention is non-transgenic and comprises the virus as defined in the third aspect of the invention, wherein the virus of the third aspect replicates within the cells of the plant, particularly, wherein the virus of the invention replicates within the cells of the plant and spreads between cells of the plant, particularly between adjacent cells of the plant and/or by the vascular system of the plant. In another particular embodiment, the plant of the eighth aspect of the invention does not comprise heterologous or artificial sequences integrated in its genome, but comprises the virus as defined in the third aspect of the invention. In a more particular, the plant of the eighth aspect of the invention does not comprise heterologous or artificial sequences integrated in its genome, but comprises the virus as defined in the third aspect of the invention, wherein the virus of the third aspect replicates within the cells of the plant, particularly, wherein the virus of the invention replicates within the cells of the plant and spreads between cells of the plant, particularly between adjacent cells of the plant and/or by the vascular system of the plant.

In a particular embodiment, plant of the eight aspect of the invention is from a genus selected from the list consisting of *Solanum sp, Arabidopsis sp., Nicotiana sp., Pyrus sp Capsicum sp., Citrullus sp., Cucumis sp., Cucurbita sp., Triticum sp., Oryza sp., Pisum sp., Cicersp., and Citrus sp.* particularly is from a species selected from the list consisting of *Solanum lycopersicum, Arabidopsis thaliana, Nicotiana benthamiana, Pyrus communis L., Solanum tuberosum, Solanum melongena, Capsicum annuum, Cucumis melo, Cucumis sativus, Citrullus lanatus, Cucurbita moschata, Zea mays, Triticum aestivum, Triticum turgidum, Oryza sativa, Pisum sativum, Cicer arietinum, Citrus sinensis, Citrus limon, Citrus paradisi and Citrus reticulata*.In another particular embodiment, the plant of the eight aspect of the invention is selected from the plants disclosed in Barathi et al. (2023) *supra,* particularly in tables 1, 2, 4-7 of Barathi et al. (2023) *supra.*

In a more particular embodiment, the plant of the invention is from a genus selected from the list consisting of *Solanum sp, Arabidopsis sp., Nicotiana sp., Pyrus sp.,* particularly from a species selected from the list consisting of *Solanum lycopersicum, Arabidopsis thaliana, Nicotiana benthamiana, Pyrus communis L..* In another particular embodiment, the plant of the invention is from a genus selected from the list consisting of *Solanum sp, Arabidopsis sp., and Nicotiana sp.,* particularly from a species selected from the list consisting of *Solanum lycopersicum, Arabidopsis thaliana, Nicotiana benthamiana.* In another particular embodiment, the plant of the invention is from the genus *Solanum sp.* particularly from the species *Solanum Lycopersicum.*

In a ninth aspect, the invention provides a plant extract from the plant of the eighth aspect of the invention.

In an embodiment of the ninth aspect, the plant extract is from a plant that comprises the virus as defined in the third aspect of the invention, particularly, from an organ of the plant that comprises said virus. In a more particular embodiment, the plant extract is from a plant organ infected with said virus.

In an embodiment, the plant extract is from a plant agroinfiltrated with the bacterium as defined in the seventh aspect, more particularly, from an organ of the plant agroinfiltrated with the bacterium as defined in the seventh aspect. In a particular embodiment, the organ of the plant from which the plant extract is obtained is a leaf of the plant, particularly from a 2-8 weeks old plant, more particularly 4-6 weeks old plant. In another particular embodiment, the organ from which the plant extract is obtained is the apical tissue, particularly the apical meristem of the plant, particularly of a 2-8 weeks old plant, more particularly of a 4-6 weeks old plant.

In another embodiment of the ninth aspect, the plant extract comprises plant tissue and a buffer, particularly in a proportion of plant tissue: buffer in g/ml of 1:50, 1:30, 1:20, 1; 15, 1:10, 1:5, 1:2, or 1:1, particularly of 1:5.

AS well understood by a skilled person, the plant tissue comprised in the plant extract of the ninth aspect is the tissue of the plant organ from which the plant extract is obtained. In another particular embodiment, the buffer comprised in the plant extract of the ninth aspect is a potassium phosphate buffer, particularly 50mM potassium phosphate, wherein the pH of the phosphate buffer is pH 6-9, particularly pH8. In an additional embodiment, the buffer further comprises polyvinylpyrrolidone 10, particularly 1% polyvinylpyrrolidone 10, and/or polyethylene glycol 6000, particularly 1% polyethylene glycol 6000. In another particular embodiment, the buffer comprises mercaptoethanol, particularly 10 mM mercaptoethanol. In an additional embodiment, the buffer of the plant extract of the ninth aspect comprises carborundum, particularly 10% (g/l) of carborundum.

In a tenth aspect, the invention provides a method for obtaining the plant as defined in the eighth aspect, the method comprising the step of:
(i) transfecting a plant with the plasmid as defined in the sixth aspect, particularly by agroinfiltration with the bacterium as defined in the seventh aspect, or alternatively,
(ii) contacting the surface of at least one organ of a plant with the virus as defined in the third aspect.

In a particular embodiment, transfecting the plant in step (i) of the method, is performed by agroinfiltration with the bacterium as defined in the seventh aspect, particularly with the bacterium of the species *Agrobacterium tumefaciens.*

In a particular embodiment of the tenth aspect, plant transfection in step (i) of the method is performed on at least one leaf of the plant, particularly on at least 4, at least 3, at least 2, or at least 1 leaves, particularly at least 1 leaf of the plant. In a more particular embodiment of the tenth aspect, the plant transfection in step (i) of the method is performed in 5, 4, 3, 2, leaves or 1 leaf, particularly 2 leaves of the plant. In another embodiment of the tenth aspect, the plants transfected with the plasmid of the sixth aspect in step (i) of the method, particularly by agroinfiltration with the bacterium of the seventh aspect, are 2-8 weeks old plant, more particularly 4-6 weeks old plant.

In an embodiment of the tenth aspect, contacting the surface of at least one organ of a plant with the virus in step (ii) of the method, comprises applying a composition that comprises the virus as defined in the third aspect of the invention, on the surface of the at least one organ of the plant. In a particular embodiment, the composition that comprises said virus, comprises the virus defined in the third aspect, and a buffer, particularly potassium phosphate buffer, particularly 50mM potassium phosphate, wherein the pH of the phosphate buffer is pH 6-9, particularly pH8. In an additional embodiment, the buffer further comprises polyvinylpyrrolidone 10, particularly 1% polyvinylpyrrolidone 10, and/or polyethylene glycol 6000, particularly 1% polyethylene glycol 6000. In another particular embodiment of the tenth aspect, the buffer of the composition of step (ii) of the method, as defined in any of the embodiments of the present paragraph, comprises mercaptoethanol, particularly 10 mM mercaptoethanol. In another particular embodiment, the buffer of the composition of step (ii) of the method, as defined in any of the embodiments of the present paragraph, comprises carborundum, particularly 10% (g/l) of carborundum.
In a particular embodiment, the composition that comprises the virus as defined in the third aspect of the invention and used in step (ii) of the method of the tenth aspect, is the plant extract as defined in the ninth aspect of the invention.

In another embodiment, the at least one organ of a plant on which the virus is applied in step (ii) of the method, as indicated in any of the embodiments of the tenth aspect of the invention, is at least one leaf, particularly, the third true leaf of an at least 1, at least 2, at least 3, at least 4, at least 5, at least 6, particularly at least 3-week-old-plants. More particularly, the at least one organ of a plant is at least one leaf, particularly, the third true leaf of a 1, 2, 3, 4, 5, 6, particularly 3, week-old-plants.

In a particular embodiment of the tenth aspect, step (ii) of the method is performed by spraying the composition comprising the virus of the invention on the plants, particularly, on the surface of the plants, more particularly, on the at least one organ of the plant, wherein said organ is as defined in the embodiments just above. In a more particular embodiment, spraying of the composition used in step (ii) of the method of the tenth aspect comprising the virus of the invention is performed on at least one leaf, particularly on at least 2 leaves, more particularly in all said leaves simultaneously. In another particular embodiment, spraying the composition used in step (ii) of the method of the tenth aspect comprising the virus of the invention in the method of the tenth aspect of the invention is performed by spraying the surface of the plant with said composition.

In a particular embodiment, the method of the tenth aspect, particularly the that comprising step (ii) of the method, is for obtaining a non-transgenic plant as defined in the eighth aspect of the invention. In another particular embodiment, the method of tenth aspect, particularly that comprising step (ii) of the method, is for obtaining a plant wherein nor the sequence of the viral vector of the first or second aspect of the invention, nor the sequence of the polynucleotide of the fifth aspect of the invention, the sequence of the plasmid of the sixth aspect of the invention is comprised or integrated in the genome of the plant. Particularly, the method for obtaining the non-transgenic plant or the plant defined in the previous embodiment comprises the steps indicated herein in the tenth aspect.

In a particular embodiment, the plant obtained from the method of the tenth aspect, particularly that comprising step (ii) of the method, is a non-transgenic plant. In another particular embodiment, the plant obtained from the method of the tenth aspect, particularly that comprising step (ii) of the method, does not comprise in its genome or integrated in its genome, nor the sequence of the viral vector of the first or second aspect of the invention, nor the sequence of the polynucleotide of the fifth aspect of the invention, nor the sequence of the plasmid of the sixth aspect of the invention.

As indicated in the eighth aspect of the invention, in a particular embodiment the plant of the invention is resistant to one or more plant pathogens. Thus, in a particular embodiment of the tenth aspect, the method of tenth aspect is for obtaining a plant resistant to one or more plant pathogens, particularly wherein the plant is as the plant resistant to one or more plant pathogens as described in the eighth aspect. Particularly, the method for obtaining a plant resistant to one or more plant pathogens comprises the steps indicated herein in the tenth aspect. In a particular embodiment, all the embodiments of the plant resistant to one or more plant pathogens of the eight aspect of the invention apply to the method for obtaining a plant resistant to one or more plant pathogens of the tenth aspect of the invention. Particularly, the one or more plant pathogens of the method for obtaining a plant resistant to one or more plant pathogens as defined herein are the one or more plant pathogens as defined in the eighth aspect of the invention.

As indicated in the eight aspect of the invention, in a particular embodiment the plant of the invention is tolerant to one or more plant pathogens. Thus, in another particular embodiment of the tenth aspect, the method of the tenth aspect is for obtaining a plant tolerant to one or more plant pathogen, particularly wherein the plant is as the plant tolerant to one or more plant pathogen as described in the eight aspect. Particularly, the method for obtaining a plant tolerant to one or more plant pathogen, comprises the steps indicated herein in the tenth aspect. In a particular embodiment, all the embodiments of the plant tolerant to one or more plant pathogen of the eight aspect of the invention apply to the method for obtaining a plant tolerant to one or more plant pathogen of the tenth aspect of the invention. Particularly, the one or more plant pathogens of the method for obtaining a plant tolerant to one or more plant pathogens as defined herein are the one or more plant pathogens as defined in the eighth aspect of the invention.

As indicated in the eight aspect of the invention, in a particular embodiment the plant of the invention is a plant with at least one improved trait, particularly one improved, more particularly as compared to a control plant. Thus, in another particular embodiment of the tenth aspect, the method is for obtaining a plant with at least one improved trait, particularly as compared to a control plant, more particularly wherein the plant with the at least one improved trait is as the plant with at least one improved trait as described in the eighth aspect of the invention. Particularly, the method for obtaining a plant at least one improved trait, particularly as compared to a control plant, comprises the steps indicated herein in the tenth aspect. In a particular embodiment, all the embodiments of the plant with at least one improved trait of the eight aspect of the invention apply to the method for obtaining a plant with at least one improved trait of the tenth aspect of the invention. In particular embodiment, the method of the tenth aspect for obtaining a plant with at least one improved trait, particularly as compared to a control plant, is for obtaining a plant with an improved trait, wherein said plant is as the plant with an improved trait defined in the eighth aspect.

Ina particular embodiment, of the tenth aspect, the method is for obtaining a plant with at least one improved trait, particularly as compared to a control plant, more particularly wherein the plant with the at least one improved trait is as the plant with at least one improved trait as described in the eighth aspect of the invention, resistant to one or more plant pathogens, particularly wherein the plant is as the plant resistant to one or more plant pathogens as described in the eighth aspect. Particularly, the method for obtaining a plant with at least one improved trait, particularly as compared to a control plant and resistant to one or more plant pathogens comprises the steps indicated herein in the tenth aspect. In a particular embodiment, plant with at least one improved trait of the eight aspect of the invention and all the embodiments of the plant resistant to one or more plant pathogens of the eighth aspect of the invention apply to the method for obtaining a plant with at least one improved trait, particularly as compared to a control plant and resistant to one or more plant pathogens. Particularly, the one or more plant pathogens of the method are the one or more plant pathogens as defined in the eighth aspect of the invention. Ina particular embodiment, the plant with at least one improved trait is a plant with one improved trait, particularly as defined in the eight aspect.

In an eleventh aspect, the invention provides a method for obtaining the plant extract according to the ninth aspect that comprises the steps of:
(i) Grinding to powder at least one organ or a section thereof of the plant as defined in the eighth aspect, and
(ii) Homogenizing the powder obtained in step (i) in a buffer.

In a particular embodiment of the eleventh aspect, the plant organ and the plant of step (i) of the method are as defined in the ninth aspect of the invention. In a particular embodiment, the plant organ or section thereof of step (i) of the method of the eleventh aspect comprises the virus as defined in the third aspect of the invention.

The term "grinding to powder", as used herein refers to reducing to powder or small fragments by friction. Methods to reduce to powder an organ of a plant are well-known by an expert in the field, and include, for instance, freezing in liquid nitrogen the plant organ, and milling the obtained product to powder.

In a particular embodiment of the eleventh aspect, the buffer of step (ii) comprises potassium phosphate buffer, particularly 50mM potassium phosphate, wherein the pH of the phosphate buffer is pH 6-9, particularly pH8. In an additional embodiment, the buffer further comprises polyvinylpyrrolidone 10, particularly 1% polyvinylpyrrolidone 10, and/or polyethylene glycol 6000, particularly 1% polyethylene glycol 6000.
In another particular embodiment, the buffer of the composition of step (ii) of the method of the eleventh aspect, as defined in any of the embodiments of the present paragraph comprises mercaptoethanol, particularly 10 mM mercaptoethanol. In another particular embodiment, the buffer of the composition of step (ii) of the method of the eleventh aspect, as defined in any of the embodiments of the present paragraph, comprises carborundum, particularly 10% (g/l) of carborundum.

In a particular embodiment, the method for obtaining the plant extract according to the ninth aspect that comprises the steps of:
(i) Grinding to powder at least one organ or a section thereof of the plant as defined in the eighth aspect, particularly at least 1 g of tissue from said at least one organ and
(ii) Homogenizing the powder obtained in step (i) in a buffer, particularly in a proportion of plant tissue: buffer in g/ml of 1:50, 1:30, 1:20, 1; 15, 1:10, 1:5, 1:2, or 1:1, particularly of 1:5.

in an additional embodiment, the method of the eleventh aspect comprises a step (iii) of filtrating the product obtained from step (ii), particularly with sterile filter paper.

### VI- Additional aspects of the invention

In a twelfth aspect, the invention provides a kit of parts comprising the viral vector as defined in the first or second aspect, the virus as defined in the third aspect, the polynucleotide as defined the fourth or fifth aspect, and/or the plasmid as defined in the sixth aspect, particularly, wherein the kit further comprises instructions for its use.

In a thirteenth aspect, the invention provides a device comprising the virus as defined in the third aspect, the bacterium as defined in the seventh aspect, or the plant extract as defined in the ninth aspect. In a particular embodiment, the device is selected from the list consisting of a syringe, a sample vial, a tube, a bag, a tissue, and a bottle.

In a fourteenth aspect, the invention provides a method for obtaining the viral vector as defined in the first or second aspect that comprises:
i) Transfecting an organ of a plant with the plasmid as defined in sixth aspect, particularly by agroinfiltration with the bacterium as defined in the seventh aspect,
ii) Allowing the plant to grow for at least 1 week, and
iii) Isolating the viral vector comprised in the organ transfected in step (i), from the plant obtained from step (ii).

In a particular embodiment of the fourteenth aspect, step (i) of the fourteenth aspect is performed as indicated in any of the embodiments of the tenth aspect for step (i) of the method of the tenth aspect. In another embodiment, the plant is allowed to grow in step (ii) of the method of the fourteenth aspect for at least 1 week, at least 2 weeks, at least 3, weeks, at least 4 weeks, at least 5 weeks, at least 6 weeks, particularly at least 2 weeks. In a more particular embodiment, the plant is allowed to grow in step (ii) of the method of the fourteenth aspect for 1-5, 2-4, 2-3 weeks, particularly, 2-3 weeks. In another embodiment, the organ of the plant is any organ as defined in the tenth aspect of the invention, particularly the apical tissue of the plant, more particularly the apical meristem of the plant.

In a fifteenth aspect, the invention provides a method for obtaining the virus as defined in the third aspect that comprises:
i) Transfecting an organ of a plant with the plasmid as defined in sixth aspect, particularly by agroinfiltration with the bacterium as defined in the seventh aspect,
ii) Allowing the plant to grow for at least 1 week, and
iii) Isolating the viral vector comprised in the organ transfected in step (i), from the plant obtained from step (ii).

In a particular embodiment, the embodiments provided above for steps (i), (ii) and (iii) of the method of the fourteenth aspect, apply to steps (i), (ii) and (iii), respectively, of the method of the fifteenth aspect.

In a sixteenth aspect, the invention provides a method for protecting a plant from one or more plant pathogens, the method comprising:
(i) transfecting a plant with the plasmid as defined in the sixth aspect, particularly by agroinfiltration with the bacterium as defined in the seventh aspect, or alternatively,
(ii) contacting the surface of at least one organ of a plant with virus as defined in the third aspect.

In a particular embodiment, the definitions and embodiments of the eighth aspect and of the tenth aspect apply to the sixteenth aspect. In a particular embodiment of the sixteenth aspect, step (i) is performed as step (i) of the method of the tenth aspect. In another particular embodiment of the sixteenth aspect, step (ii) is performed as step (ii) of the method of the tenth aspect

In a seventeenth aspect, the invention provides a method for the treatment of one or more plant pathogens diseases in a plant, the method comprising:
(i) transfecting a plant with the plasmid as defined in the sixth aspect, particularly by agroinfiltration with the bacterium as defined in the seventh aspect, or alternatively,
(ii) contacting the surface of at least one organ of a plant with the virus as defined in the third aspect.

In a particular embodiment of the seventeenth aspect, contacting the surface of at least one organ of a plant with the virus as defined in the third aspect in step (ii) of the method, comprises applying a composition that comprises said virus on the surface of the at least one organ of the plant. In a particular embodiment, the composition comprising the virus is the plant extract as defined in the ninth aspect of the invention.

In a particular embodiment of the seventeenth aspect, step (i) is performed as step (i) of the method of the tenth aspect. In another particular embodiment of the seventeenth aspect, step (ii) is performed as step (ii) of the method of the tenth aspect

The term "treating one or more plant pathogens diseases", as used herein, refers to reducing the concentration of the one ore more plant pathogens in a plant, particularly, in a cell of a plant, or at least reducing the symptoms and the damage caused by said one or more plant pathogens in a plant. In an embodiment, the embodiments and definitions provided in the eighth aspect of the invention apply to the seventeenth aspect of the invention. In a particular embodiment, the embodiments and definitions provided in the eighth aspect of the invention for the plant resistant to one or more plant pathogens and to the plant tolerant to one or more plant pathogens, apply to the seventeenth aspect of the invention. In a more particular embodiment, treating a plant pathogen disease comprises reducing the concentration of the one or more plant pathogens in a plant, particularly in an organ of the plant exposed to the plant pathogen plant, more particularly in the cells of said organ, in a percentage as that indicated in the eighth aspect for the plant resistant to the said plant pathogens. In another particular embodiment, treating one or more plant pathogens disease comprises reducing the level of symptoms associated to one or more plant pathogens in a plant, in a percentage as that indicated in the eighth aspect for the plant tolerant to one or more plant pathogens, even when maintaining the plant pathogens concentration in the plant organ exposed to said pathogens in a percentage as that indicated in the eighth aspect for the plant resistant tolerant to one or more plant pathogens. The definitions and embodiments of the tenth aspect also apply to the method of the seventeenth aspect.

In an eighteenth aspect, the invention provides a method for improving a trait of a plant, the method comprising:
(i) transfecting a plant with the plasmid as defined in the sixth aspect, particularly by agroinfiltration with the bacterium as defined in the seventh aspect, or alternatively,
(ii) contacting the surface of at least one organ of a plant with the virus as defined in the third aspect.

In a particular embodiment of the eighteenth aspect, contacting the surface of at least one organ of a plant with the virus as defined in the third aspect in step (ii) of the method, comprises applying a composition that comprises said virus on the surface of the at least one organ of the plant. In a particular embodiment, the composition comprising the virus is the plant extract as defined in the ninth aspect of the invention.

In a particular embodiment of the eighteenth aspect, step (i) is performed as step (i) of the method of the tenth aspect. In another particular embodiment of the eighteenth aspect, step (ii) is performed as step (ii) of the method of the tenth aspect

In a particular embodiment, the plant trait improved with the method of the eighteenth aspect of the invention is as the improved trait indicated in the eighth aspect of the invention for the plant with an improved trait. In another particular embodiment, the trait improved with the method of the eighteenth aspect of the invention is selected from the improved traits indicated in the eighth aspect of the invention, particularly is selected from the list consisting of crop yield, shelf life of fruits, shelf life of vegetables, nutritional value, organoleptic properties, abiotic stress resistance (for instance, to drought, salinity, high temperatures, or cold), or mal sterility. In a particular embodiment, the trait improved with the method of the eighteenth aspect of the invention is selected from those disclosed in tables 1 and in table 7 of Barathi et al., (2023), *supra.*

In a particular embodiment of the eighteenth aspect, improving a trait of a plant comprises, particularly consists of, increasing the level of said trait in the plant in at least 10%, 20%, 30%, 40%, 50%, 75%, 90%, 95%, 100%, 150%, 200%, 250%, 300%, 350%, 400%, particularly in at least 20%, as compared to a control plant. In a more particular embodiment of the eighteenth aspect, improving a trait of a plant comprises, particularly consists of, increasing the level of said in the plant in at least 50% as compared to a control plant. In a yet more preferred embodiment of the eighteenth aspect, improving a trait of a plant comprises, particularly consists of, increasing the level of said in the plant in at least 100%, as compared to a control plant.

In a particular embodiment, the control plant is as the control plant indicated in the eighth aspect of the invention for the plant with an improved trait as compared to a control plant.

In a particular embodiment, the embodiments of the eight and tenth aspect of the invention apply to the eighteenth aspect of the invention.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention.

The following examples and drawings are provided by way of illustration, and they are not intended to be limiting of the present invention. Reference signs related to drawings and placed in parentheses in a claim, are solely for attempting to increase the intelligibility of the claim, and shall not be construed as limiting the scope of the claim. Furthermore, the present invention covers all possible combinations of particular and preferred embodiments described herein.

### Examples

### Example 1 - Materials and methods

### Plant materials and growth conditions

*Nicotiana benthamiana* and *Arabidopsis thaliana* Col-0 plants were grown in a growth chamber at 25°C with a 12 h-light/12 h-dark photoperiod or at 22°C with a 16 h-light/8 h-dark photoperiod, respectively. Arabidopsis Col-0 plants were genetically transformed using the floral dip method (Clough,S.J. et al., (1998) Floral dip: a simplified method for Agrobacterium-mediated transformation of Arabidopsis thaliana". Plant J, 16, 735-43) with the *Agrobacterium tumefaciens* GV3101 strain (for example from Goldbiotechnology^{®}). T1 transgenic Arabidopsis were grown as previously (L6pez-Dolz,L., Spada,M. et al., (2020) Fine-tune control of targeted RNAi efficacy by plant artificial small RNAs. Nucleic Acids Res, 48, 6234-6250). Photographs were taken with a Nikon D3000 digital camera with AF-S DX NIKKOR 18-55 mm f/3.5-5.6G VR lens.

### Plant phenotyping

Plant phenotyping analyses were conducted in blind as described (Lopez-DoIz,L., et al., supra). Briefly, the flowering time of each independent line is the number of days elapsed from seed plating to first bud opening (or "days to flowering"). The "CH42" phenotype was scored in 10 days old seedlings and was reported as "weak", "intermediate" or "severe" if seedlings had more than two leaves, exactly two leaves or no leaves at all (only two cotyledons), respectively.

### DNA constructs

35S:*AtTAS1c*-based syn-tasiRNA constructs were described before (Lopez-DoIz,L., et al., supra). To generate 35S:AtMIR173TS-based constructs and *35S:NbmiR482aTS-NbSu* specific ssDNA oligos were annealed and inserted in *pENTR-D-TOPO* following manufacturer's instructions (Invitrogen); syn-tasiRNA cassettes were transferred by LR recombination to *pMDC32..* To generate *35S:AtTAS1c(NbmíR482aTS)-NbSu,* target site was introduced by mutagenic PCR using the *pENTR-AtTAS1c-D2-NbSu* construct as template with oligos AC-631 and AC-505; syn-tasiRNA cassette was transferred by LR recombination to *pMDC32.* To generate PVX-based or TRV-based syn-tasiRNA constructs the corresponding annealed oligos were ligated into the PVX and TRV plasmids, previously digested with *Mlu*I and *Bsa*I, respectively, as indicated below. Oligonucleotides used to make DNA constructs are listed in Table 1.

**Table 1. Name, sequence and use of DNA oligonucleotides used in this study.**

| **Oligonucleotide** | **Sequence** | **SEQ ID NO.** | **Construct/Aim** |
|---|---|---|---|
| AC-55 | AGGGGCCATGCTAATCTTCTC | 32 | DNA probe for U6 detection |
| AC-157 | GGCCTCTTCCTTTATAACCAA | 33 | DNA probe for syn-tasiR-AtFT detection |
| AC-158 | AGGGATTTCCGTGACACTTAA | 34 | DNA probe for syn-tasiR-AtCH42 detection |
| AC-159 | AAAAATGGCTGAGGCTGATGA | 35 | qPCR amplification of *AtACT2* mRNA |
| AC-160 | GAAAAACAGCCCTGGGAGC | 36 | |
| AC-163 | CATGCACAAGTAGGGACGGTT | 37 | qPCR amplification of *AtCH42* mRNA |
| AC-164 | GTCACGGAAATCCTTTGGGTT | 38 | |
| AC-169 | TGGAACAACCTTTGGCAATG | 39 | qPCR amplification of *AtFT* mRNA |
| AC-170 | CGACACGATGAATTCCTGCA | 40 | |
| AC-355 | GACCCTGATGTTGATGTTCGCT | 41 | qPCR amplification of *NbSu* mRNA |
| AC-356 | GAGGGATTTGAAGAGAGATTTC | 42 | |
| AC-365 | GACCCTGATGTTGATGTTCGCT | 43 | PCR&qPCR amplification of *NbPP2A* mRNA |
| AC-366 | GAGGGATTTGAAGAGAGATTTC | 44 | |
| AC-417 | G+CGG+GAA+GTC+CAC+CAC+GG T+TA | 45 | LNA probe for amiR-NbSu detection |
| AC-505 | CCCCATACCACAACAAAACAATGC TGTTTCATCTCG | 46 | *pENTR-AtTAS1c(NbmiR482aTS)-NbSu* |
| AC-518 | cacttacccgagttaacgccAAACCTAAAC CTAAACGG | 47 | *35S:TRV2-AtTAS1c(NbmiR482aTS)-D2-NbSu-2* |
| AC-519 | gtttaatgtcttcgggacatATTTCACTTTAC GATGTGG | 48 | |
| AC-617 | cacttacccgagttaacgccAGTAGAGAAG AATCTGTA | 49 | *35S:TRV2-amiR-GUSlamiR-TSWV* |
| AC-618 | gtttaatgtcttcgggacatAGTAAGAAGAG CCAA | 50 | |
| AC-631 | GGAGTCGGGAATAGACCATTTAT GTATGACTCCCGG | 51 | *pENTR-AtTAS1c(NbmiR482aTS)-NbSu* |
| AC-641 | CACCGTGGTATGGGGGGAGTCGG GAATAGACCATTTATGTATGACTC CCGGAATTCCA | 52 | *pENTR*/*pMDC32-NbmiR482a-NbSu* |
| AC-642 | TGGAATTCCGGGAGTCATACATAA ATGGTCTATTCCCGACTCCCCCCA TACCACGGTG | 53 | |
| AC-666 (duplex) | agaggtcagcaccagctagcGTGGTATGG GGGGAGTCGGGAATAGACCATTT ATGTATGACTCCCGGAATTCCAag ggtttgttaagtttccct | 54 | *35S:PVX-NbmiR482aTS-NbSu-2* |
| AC-667 (duplex) | cacttacccgagttaacgccGTGGTATGG GGGGAGTCGGGAATAGACCATTT ATGTATGACTCCCGGAATTCCAatg tcccgaagacattaaac | 55 | *35:TRV-NbmiR482aTS-NbSu-2* |
| AC-676 | CACCGTGATTTTTCTCTACAAGCG AATAGACCATTTATGCGCTTGCTG AGTTTCCCCC | 56 | *pENTR*/*pMDC32-AtMIR173TS-GUS(At)* |
| AC-677 | GGGGGAAACTCAGCAAGCGCATA | 57 | |
| | AATGGTCTATTCGCTTGTAGAGAA AAATCACGGTG | | |
| AC-678 | CACCGTGATTTTTCTCTACAAGCG AATAGACCATTTATTAAGTGTCAC GGAAATCCCT | 58 | *pENTR*/*pMDC32-AtMIR173TS-AtCH42* |
| AC-679 | AGGGATTTCCGTGACACTTAATAA ATGGTCTATTCGCTTGTAGAGAAA AATCACGGTG | 59 | |
| AC-680 | CACCGTGATTTTTCTCTACAAGCG AATAGACCATTTATTGGTTATAAA GGAAGAGGCC | 60 | *pENTR*/*pMDC32-AtMIR173TS-AtFT* |
| AC-681 | GGCCTCTTCCTTTATAACCAATAA ATGGTCTATTCGCTTGTAGAGAAA AATCACGGTG | 61 | |
| AC-682 | CACCGTGATTTTTCTCTACAAGCG AATAGACCATTTATTGGTTATAAA GGAAGAGGCCTCCCATTCGATAC TGCTCGCC | 62 | *pENTR*/*pMDC32-AtMIR173TS-AtFT-AtTRY* |
| AC-683 | GGCGAGCAGTATCGAATGGGAGG CCTCTTCCTTTATAACCAATAAATG GTCTATTCGCTTGTAGAGAAAAAT CACGGTG | 63 | |
| AC-684 | CACCGTGATTTTTCTCTACAAGCG AATAGACCATTTATCCCATTCGAT ACTGCTCGCCTTGGTTATAAAGGA AGAGGCC | 64 | *pENTR*/*pMDC32-AtMIR173TS-AtTRY-AtFT* |
| AC-685 | GGCCTCTTCCTTTATAACCAAGGC GAGCAGTATCGAATGGGATAAAT GGTCTATTCGCTTGTAGAGAAAAA TCACGGTG | 65 | |
| AC-775 (duplex) | agaggtcagcaccagctagcGGCATGGG CGGTGTAGGCAAGATAGACCATTT ATCCCCCTTGTATGATTCCCGCTC TCAATGTCGATTGTCCCAGagggtttg ttaagtttccct | 66 | *35S:PYX-SlymiR482b-SlySu-1-SlySu-2* |
| AC-824 | TGTAGGCATGGGCGGTGTAGGCA AGATAGACCATTTATATTGACCCA CACTTTGCCGA | 67 | *pMDC32B-SlymiR482bTS-GUS* |
| AC-825 | AATGTCGGCAAAGTGTGGGTCAA TATAAATGGTCTATCTTGCCTACA CCGCCCATGCC | 68 | |
| AC-826 | TGTAGGCATGGGCGGTGTAGGCA AGATAGACCATTTATGTATGACTC CCGGAATTCCA | 69 | *pMDC32B-SlymíR482b TS-NbSu* |
| AC-827 | AATGTGGAATTCCGGGAGTCATAC ATAAATGGTCTATCTTGCCTACAC CGCCCATGCC | 70 | |
| AC-654 | GGGAATCAATCACAGTGTTGGC | 71 | |
| AC-655 | GCTACTATGGCACGGGCTGTAC | 72 | |
| AC-523 | TCGGTTTGCTGACCTACTGG | 73 | |
| AC-524 | AACCTAAAACTTCAGACACGG | 74 | |

### Protocol to generate PVX-based or TRV-based syn-tasiRNA constructs

### 1. Preparation of the dsDNA amiRNA insert

Design of the sequence of the insert was made as follows and ordered as a dsDNA (eg. ultramer duplex in IDT):
A- For PVX-based constructs: 
agaggtcagcaccagctagcXXXXXXXXXXXXXXXXXXXXXXTAGACCATTTAX1X2X3X4X5X6X7X8X9X10X11
X12X13X14X15X16X17X18X19X20X21agggtttgttaagtttccct (SEQ ID NO. 75)
 Where:
   - x (lower cases) is a DNA base of the PVX sequence, required for Gibson-based assembly
   - X (regular) is a DNA base of a 22-nt miRNA target site
   - **X** (bold underlined) is a DNA base of the *AtTAS1c*-based spacer
   - ***X*** (bold italics) is a DNA base of the syn-tasiRNA sequence, and the subscript number is the base position in the syn-tasiRNA-1 21-mer
B- For TRV-based constructs: 
cacttacccgagttaacgccXXXXXXXXXXXXXXXXXXXXTAGACCATTTAX1X2X3X4X5X6X7X8X9X10X11
X12X13X14X15X16X17X18X19X20X21atgtcccgaagacattaaac (SEQ ID NO.76)
 Where:
   - x (lower cases) is a DNA base of the TRV sequence, required for Gibson-based assembly
   - X (**regular**) is a DNA base of a 22-nt miRNA target site
   - **X** (bold underlined) is a DNA base of the *AtTAS1c*-based spacer
   - ***X*** (bold italics) is a DNA base of the syn-tasiRNA sequence, and the subscript number is the base position in the syn-tasiRNA-1 21-mer

Fragment #1 (insert) is ready.

### 2. Preparation of the vector

A- For PVX-based constructs:
   - Digest the PVX plasmid with Mlul.
   - Gel purify the 9921 bp band.
   - Quantify 1 ul in Nanodrop.
B- For TRV-based constructs:
   - Digest the TRV plasmid with Bsal.
   - Gel purify the 5818 bp band.
   - Quantify 1 ul in Nanodrop.

Fragment #2 (backbone vector) is ready.

### 3. Assembly

- Assemble the Gibbson reaction as described below:
   - Fragment 1 (dsDNA insert)^{a}
   - Fragment 2 (vector)^{b,c,d}
   - GeneArt Gibson Assembly HiFI Master Mix 5 µL
   - dH2O to 10 µL
   - Total volume: 10 µL

   a:The optimal amount of vector is between 50-100 ng
   b: Insert/vector molar excess is between 2-3.
   c:Total DNA amount is between 0.02-0.5 pmol
   d:Mass to moles conversions can be calculated here:
   http://nebiocalculator.neb.com/#!/ssdnaamt
- Incubate reactions at 50°C for 1h.
- Clean up reactions with a column (e.g. Zymo Research)
- Transform 1-4 µL in E. coli DH5a
- Plate in L-Kan plates and incubate 16h at 37°C

### 4. Clone verification

- Pick several colonies and grow in liquid LB-Kan 16h at 37°C, and purify plasmids.
- For PVX-based constructs:
   - Digest candidate clones with Apal+Xhol
      Good clones: 8595 + 1409 bp
      Bad clones (empty-PVX-Mlul plasmid): 8595 + 1738 bp
   - Confirm insert sequence by Sanger sequencing with forward and reverse oligos AC-654 (GGGAATCAATCACAGTGTTGGC) and/or AC-655 (GCTACTATGGCACGGGCTGTAC), respectively.
   - For TRV-based constructs:
      Digest candidate clones with Bpil
      Good clones: 2296 + 1064 + 1011 + 728 + 423 + 378 +3 bp
   - Confirm insert sequence by Sanger sequencing with forward and reverse oligos AC-523 (TCGGTTTGCTGACCTACTGG) and/or AC-524 (AACCTAAAACTTCAGACACGG), respectively.

### Transient expression of constructs and virus infection assays

DNA constructs were agroinfiltrated in *N. benthamiana* leaves as described (Cuperus,J.T. et al., (2010) Unique functionality of 22-nt miRNAs in triggering RDR6-dependent siRNA biogenesis from target transcripts

in Arabidopsis. Nat Struct Mol Biol, 17, 997-1003), using A. *tumefaciens* GV3101 strain. Infection assays with TSWV LL-N.05 isolate were done as described (Carbonell,A., et al., (2019) Fast-Forward Identification of Highly Effective Artificial Small RNAs Against Different Tomato spotted wilt virus Isolates. Mol Plant Microbe Interact, 32, 142-156).

### RNA preparation

Total RNA from *N. benthamiana* leaves or from *A*. *thaliana* seedlings was isolated as follows. Tissue was ground in liquid nitrogen to fine powder and homogenized in extraction buffer (1 M guanidinium thiocyanate, 1 M ammonium thiocyanate, 0.1 M sodium acetate, 5 % glycerol, 38 % water-saturated phenol). Next, RNA was extracted by chloroform extraction and precipitated after adding 0.5 volumes of isopropanol for 20 min. Triplicate samples from pools of two *N. benthamiana* leaves or 9-12 Arabidopsis seedlings were analyzed.

### Real-time RT-qPCR

cDNA was obtained from 500 ng of DNasel-treated total RNA from apical leaves at 14 dpa, using the PrimeScript RT Reagent Kit (Perfect Real Time) (Takara) according to the manufacturer's instructions.

Real time RT-qPCR was done as described in a QuantStudio 3 Real-Time PCR system (Thermo Fisher Scientific). *NbSu* and *AtFT*/*AtCH42* target RNA expression levels were calculated relative to *N. benthamiana PROTEIN PHOSPHATASE 2A (NbPP2A)* and Arabidopsis *ACTIN 2* (*AtACT2*) reference genes, respectively, using the delta delta cycle threshold comparative method of the QuantStudio Design and Analysis Software (version 1.5.1.; Thermo Fisher Scientific). Oligonucleotides used for RT-qPCR are listed in Table 1 above.

### Small RNA blot assays

Small RNA blot assays and band quantification from radioactive membranes were done as described (Cisneros,A.E., et al., (2022) Systemic silencing of an endogenous plant gene by two classes of mobile 21-nucleotide artificial small RNAs. Plant J, 110, 1166-1181). Oligonucleotides used as probes for sRNA blots are listed in Table S1.

### Small RNA sequencing and data analysis

Total RNA quantity, purity and integrity was analyzed with a 2100 Bioanalyzer (RNA 6000 Nano kit, Agilent) and submitted to BGI (Hong Kong, China) for sRNA library preparation and SE50 sequencing in a DNBSEQ-G-400 sequencer (BGI). After reception of quality-trimmed, adaptor removed clean reads from BGI, fastx_collapser was used to collapse identical reads into a single sequence, while maintaining read counts. A custom Python script was then used to map each clean, unique read against the forward strand of the syn-tasiRNA precursor overexpressed in each sample not allowing mismatches or gaps, and also to calculate the counts and RPMs (reads per million mapped reads) for each mapping position. Processing accuracy of syntasiRNA transcripts was assessed by quantifying the proportion of 19-24 nucleotide sRNA (+) reads that mapped within ± 4 nucleotides of the DCL4 processing position 3'D2 [+] as reported previously (Cuperus,J.T., et al., supra; Cisneros,A.E., et al., supra). Phasing register tables were built by calculating the proportion of 21-nucleotide sRNA (+) reads in each register relative to the 22-nt miRNA cleavage site for all 21 nucleotide positions downstream of the cleavage site, as described previously (Carbonell,A., et al., (2014) New generation of artificial MicroRNA and synthetic trans-acting small interfering RNA vectors for efficient gene silencing in Arabidopsis. Plant Physiol, 165, 15-29).

### Gene and virus identifiers

Arabidopsis and *N. benthamiana* gene identifiers are *AtACT2* (AT3G18780), *AtCH42* (AT4G18480), *AtFT* (AT1G65480), *NbSu* (Nbv5.1tr6204879), *NbPP2A* (Nbv5.1tr6224808) and SISu (Solyc10g008740). TSWV LL-N.05 segment L, M and S genome identifiers are KP008128, FM163373 and KP008129, respectively. PVX sequence variant MT799816.1 was cloned into a derivative of *pLX-B2* (KY825137.1). For TRV see Aragonés,V., et al., (2022) Simplifying plant gene silencing and genome editing logistics by a one-Agrobacterium system for simultaneous delivery of multipartite virus vectors. Biotechnology Journal, n/a, 2100504.

### Example 2 - Results

### 1. SILENCING BY SYN-TASIRNAS DERIVED FROM MINIMAL PRECURSORS

### 1.1 in Arabidopsis thaliana

Syn-tasiRNAs are typically expressed from Arabidopsis *TAS* precursors, such as *AtTAS1c* or *AtTAS3.* To test whether *AtTAS1c* sequences upstream AtmiR173 target site (TS) and dowstream the syn-tasiRNA could be dispensable for efficient syn-tasiRNA biogenesis, several syn-tasiRNA constructs were generated to express a syn-tasiRNA targeting Arabidopsis *FLOWERING LOCUS T* (*AtFT*) or *CHLORINE42 (AtCH42)* (**Fig. 1A**) for inducing late flowering or bleaching, respectively. Two minimal syn-tasiRNA constructs were generated including only the AtMIR173 TS, a 11 bp spacer derived from *AtTAS1c* and a syn-tasiRNA (syn-tasiR-AtFT or syn-tasiR-At-CH42) (**Fig. 1B**). Both constructs were introduced in Arabidopsis and T1 lines were monitored for silencing phenotype appearance. For comparative purposes constructs expressing both syn-tasiRNAs from the full-length *AtTAS1c* sequence (**Fig. 1B**) were also transformed into Arabidopsis, as well as constructs expressing the syn-tasiR-GUS sequence from full-length or minimal precursors. Minimal precursors induced severe silencing phenotypes, similarly to full-length precursors (**Fig. 1C**). Target mRNA levels were also significantly reduced to similar levels when syn-tasiRNAs were expressed from full-length or minimal precursors. Finally, processing accuracy of full-length and minimal precursors was also similar (**Fig. 1E**).

Next, it was analyzed if two different syn-tasiRNA sequences could be produced from a single minimal precursor. Thus, two constructs were generated to express syn-tasiR-AtFT and syn-tasiR-AtTRY (**Fig. 2A**) to silence *ATFT* or *AtTRY* and induce late flowering and increase trichome number, respectively, from positions D2 and D3 or viceversa (**Fig. 2B**). These constructs were introduced in *A*. *thaliana* and T1 plants were monitored for silencing phenotype appearance. It was observed that plants expressing syn-tasiR-AtFT and syn-tasiR-AtTRY displayed the double silencing phenotype of late flowering and increased trichome number, in a precursor- and position-independent manner (**Fig. 2C**). Target mRNA levels were similarly reduced in plants expressing both syn-tasiRNAs in any of the two configurations and from full-length or minimal precursors (**Fig. 2D**). Finally, both classes of precursors were accurately processed and accumulated phased syn-tasiRNAs (**Fig. 2E**).

### 1.2 in Nicotiana benthamiana

Syn-tasiRNA biogenesis from the syn-tasiRNA precursor (*AtTAS1c*) requires miR173, a miRNA only present in Arabidopsis (not in *N. benthamiana* or other plant species). Moreover, *AtTAS1c* transcript is 1044 nt long, which may result in an unstable insert when introduced in viral vectors for syn-tasiRNA expression. Thus, it was necessary to i) replace miR173 target site with a target site from an endogenous *N. benthamiana* 22-nt miRNA to trigger syn-tasiRNA biogenesis, and ii) reduce as much as possible *AtTAS1c* precursor length. The results showed that fully functional syn-tasiRNAs can be generated *in planta* from constructs expressing minimal precursors only including the sequence of the target site of an endogenous 22-nt miRNA trigger upstream of the syn-tasiRNA sequence (**Fig. 3**). In particular, *Su* silencing was induced in leaves agroinfiltrated with a minimal syn-tasiRNA construct expressing a target site sequence that fully base-pairs with endogenous *N. benthamiana* miR482a miRNA (miR482TS) followed by the sequence of syn-tasiR-Su, a syn-tasiRNA with the same sequence than amiR-Su (**Fig. 3A**). Similar results have been obtained using an artificial target site that fully base-pairs with endogenous NbmiR6019.

### 1.3 in Solanum lycopersicum

The same approach presented for *N. benthamiana* was followed to test if syn-tasiRNA biogenesis could be triggered in a crop such as tomato (*Solanum lycopersicum*)*.* A construct was generated to express syn-tasiR-NbSu from a minimal precursor including SlmiR482b TS, which should be recognized by endogenous 22-nt miR482b (**Fig. 4A**). As a negative control construct we also generated a similar minimal precursor construct to express syn-tasiR-GUS (**Fig. 4A**). Both constructs were agroinfiltrated in *S*. *lycopersicum* leaves together with a positive control construct expressing the same syn-tasiRNA from an *AtTAS1c*-based construct also expressing AtmiR173, and syn-tasiR-NbSu accumulation was analyzed by northern blot at 2 days post-agroinfiltration. As shown in **Fig. 4B****,** syn-tasiR-NbSu accumulated in leaves expressing the *35S:SlmiR482b-NbSu* minimal construct, similarly to when expressed from the positive control construct.

### 1.4. Conclusions

In summary, syn-tasiRNA biogenesis can be triggered from endogenous plant precursors including a target site cleaved by a 22-nt endogenous miRNA. Because such type of precursors are present in all plant lineages, this discovery allows the possibility of expressing syn-tasiRNAs in virtually any plant species.

### 2. Expression of syn-tasiRNAs through viral vectors (svn-tasiR-VIGS)

### 2.1 Silencing of endogenous plant genes

### a. TRV-based syn-tasiR-VIGS

One possible advantage of a minimal syn-tasiRNA precursor could be its higher stability when inserted into a viral vector. To test this hypothesis, it was inserted into a TRV vector (Aragonés,V., et al., supra) a minimal syn-tasiRNA precursor including NbmiR482aTS, the 11 bp *AtTAS1c* spacer and the syn-tasiR-NbSu sequence aimed to silence *NbSu,* thus resulting in the *35S:TRV2-NbmiR482aTS-NbSu* construct (**Fig. 5A**). It was also generated the *35S:TRV2-AfTAS1c(NbmiR482aTS)-NbSu* construct for expressing syn-tasiR-NbSu from the *AtTAS1c* full-length precursor embedded into TRV (**Fig. 5A**). These constructs together with the control *35S:TRV2* not expected to induce any bleaching phenotype were coagroinoculated in *N. benthamiana* plants with the *35S:TRV1* construct, containing other TRV sequences necessary for the infection. At 7 days post agroinoculation (dpa) plants agroinoculated with *35S:TRV2-NbmiR482aTS-NbSu* started to display bleaching areas in apical leaves. At 14 dpa, apical leaves were clearly bleached, accumulated low levels of *NbSu* mRNA (**Fig. 5C**) and high levels of syn-tasiR-NbSu (**Fig. 5E**), in contrast with the apical leaves of plants coagroinoculated with *35S:TRV2-AtTAS1c(NbmiR482a)-NbSu* or *35S:TRV2* (**Fig. 5B**) that were green, accumulated *NbSu* mRNA levels similar to control plants and did not accumulate syn-tasiR-NbSu. Interestingly, at this time-point full-length syn-tasiRNA precursors were not detected by RT-PCR, while bands corresponding to minimal precursors were present (**Fig. 5D**). Sanger sequencing of minimal syn-tasiRNA RT-PCR products revealed no sequence alterations. These results show that minimal but not *AtTAS1c*-based syn-tasiRNA precursors can produce functional syn-tasiRNAs to silence endogenous genes when expressed from TRV-based viral vectors.

### b. PVX-based syn-tasiR-VIGS

Next, it was tested if syn-tasiRNAs could be efficiently expressed from a different viral vector, such as Potato virus X. Thus, the full-length *AtTAS1c* precursor and the minimal syn-tasiRNA precursor were inserted independently into PVX in *35S:PVX* to generate *35S:PVX-AtTAS1c(NbmiR482aTS)-NbSu* and *35S:PVX-NbmiR482aTS-NbSu,* respectively (**Fig. 6A**). Both constructs were agroinoculated in *N. benthamiana* leaves together with the *35S:PVX* control. At 7 dpa, bleaching areas started to grow in plants agroinoculated with *35S:PVX-NbmiR482aTS-NbSu.* At 14 dpa, intense bleaching was observed in the apical leaves of these plants, while weak bleaching was noticed in some apical leaves from plants agroinoculated with *35S:PVX-AfTAS1c(NbmiR482aTS)-NbSu* (**Fig. 6B**). Northern blot analysis revealed high levels of syn-tasiR-NbSu in apical leaves of plants agroinoculated with *35S:PVX-NbmiR482aTS-NbSu,* while this same syn-tasiRNA was barely detected in apical leaves from plants agroinoculated with *35S:PVX-AtTAS1c(NbmiR482aTS)-NbSu* (**Fig. 6C**). These results extend the results observed with TRV and reinforce the concept that minimal syn-tasiRNA precursors offer an advantage over full-length *AtTAS1c*-based precursors for producing high levels of syn-tasiRNAs for highly specific gene silencing in plants.

Next, it was tested if syn-tasiR-VIGS could be used in a crop such as tomato to silence endogenous genes. Thus, the *35S:PVX-SlmiR482bTS-SlSu-1-SlSu-2* construct was made to express 2 different syn-tasiRNAs (syn-tasiR-SlSu-1 and syn-tasiR-SlSu-2) -designed to silence specifically tomato *SULPHUR* gene (*SlSu*)-from a minimal precursor including the artificial SlmiR482b target site described in Fig. 4 in PVX (**Fig. 7A**). Plants were agroinoculated with this construct together with the empty PVX control *35S:PVX.* At 14 dpa some of the apical leaves from plants agroinoculated with *35S:PVX-SlmiR482bTS-SlSu-1-SlSu-2* displayed obvious bleaching contrary to leaves of similar age from control plants. Importantly, syn-tasiRNA accumulation in PVX-infected apical leaves was confirmed by northern blot (**Fig. 7B**).

### c) Conclusions

These are the first reported results of syn-tasiR-VIGS in plants. These results indicate that that i) syn-tasiRNAs can be expressed from RNA viruses to efficiently silence host genes in different plant species, and ii) the length of the syn-tasiRNA precursor inserted in the viral genome is critical, as no efficient silencing is induced by syn-tasiRNAs derived from full-length AtTAS1c-based precursors.

### 2.2 Silencing of pathogenic RNAs: viral vector-based vaccines expressing artificial small RNAs for antiviral crop protection

Next, it was tested whether the viral vector could also be used to target exogenous RNAs, such as those from pathogenic RNA viruses to induce antiviral resistance.

Here, it was generated a TRV-based construct to express the four anti-TSWV (Tomato spotted wilt virus) syn-tasiRNAs from a minimal precursor including NbmiR6019a target site (*35S:TRV2-syn-fasiR-TSWV(x4)*) (**Fig**. **8**). For comparative purposes it was also generated a *35S:TRV2-amiR-TSWV* construct to express a single amiRNA (amiR-TSWV) against TSWV, (**Fig. 8**). As a negative control it was included the *35S:TRV2-amiR-GUS* construct to express an amiRNA against GUS (amiR-GUS) that should not have any protective effect against TSWV. Constructs were agroinoculated in *N. benthamiana* leaves from independent plants, and 5 days later, the same leaves were mechanically inoculated with a crude extract from TSWV-infected plants. At 9 dpa, all three plants agroinoculated with *35S:TRV2-amiR-GUS* showed intense TSWV symptoms, while all the plants agroinoculated with *35S: TRV2-syn-fasiR-GUS(x4)* were symptomless. The three plants agroinoculated with *35S:TRV2-amiR-TSWV* showed very mild viral symptoms. Later, at 16 dpa, all three plants agroinoculated with *35S:TRV2-syn-fasiR-TSWV(x4)* displayed mild TSWV symptomatology, while plants agroinoculated with *35S:TRV2-amiR-TSWV* or *35S:TRV2-amiR-GUS* showed intermediate and severe symptoms, respectively. These results show that TRV-based syn-tasiR-VIGS interfere with TSWV infection, and more than TRV-based amiR-VIGS. Importantly, they surprisingly show that plants can be vaccinated with a viral vector expressing multiple syn-tasiRNA against a pathogenic virus, and this vaccination protects the plant against this pathogenic virus.

## Claims

1. A viral vector comprising a heterologous nucleotide sequence that encodes or corresponds to an RNA sequence S1 that has a length of 52-350 ribonucleotides and that comprises regions R1, R2 and R3 directly linked to each other as indicated in formula (i):
(i) 5'-R1-R2-R3-3',
wherein:
- region R1 consists of the target site of a sRNA,
- region R2 consists of an RNA sequence of 11 ribonucleotides, and
- Region R3 consists of the RNA sequence of one or more siRNAs, wherein when R3 is formed by more than one siRNA sequence, the siRNA sequences are directly linked to each other.

2. The viral vector according to claim 1, wherein the sum of the lengths of all the heterologous sequences comprised in the viral vector is equal to less than 400 nucleotides.

3. The viral vector according to any one of claims 1-2 wherein the one or more siRNAs have a length of 22 nucleotides, and/or region R2 consists of the sequence SEQ ID NO. 1 (UAGACCAUUUA) or a variant thereof at least 60% identical to SEQ ID NO.1

4. The viral vector according to any one of claims 1-3 wherein the RNA sequence S1 is operatively linked to an expression promoter.

5. The viral vector according to any one of claims 1-4, wherein the one or more siRNAs target one or more genes in the genome of one or more pathogens of a plant, and/or one or more genes in the genome of a plant, wherein the one or more genes in the genome of one or more pathogens of the plant are genes encoding a protein selected from the list consisting of the viral coat protein (CP), the viral movement protein (MP), RNA polymerases, the viral RNA-dependent RNA polymerase (RdRp), the viral suppressors of RNA silencing (VSRs) and combinations thereof, and the one or more genes in the genome of the plant are selected from the list consisting of SFT (single flower truss), TFL1 (terminal flower 1), DET1 (de-etiolated 1), ALS (acetolactate synthase), ACCase (acetyl-CoA carboxylase), EPSPS (5-enolpyruvylshikimate-3-phosphate) and combinations thereof.

6. The viral vector according to claim 5 wherein the one or more plant pathogens are selected from the group consisting of viruses, viroids, bacteria, fungi, insects, nematodes and combinations thereof, wherein the viruses are particularly selected from the group consisting of Tomato spotted wilt virus (TSWV), Tomato brown rugose fruit virus (TOBRFV), Tomato leaf curl new delhi virus (ToLCNDV) and combinations thereof, and the viroids consist of *Potato spindle tuber viroid* (PSTVd).

7. The viral vector according to any one of claims 5-6, wherein the sRNA is expressed in the plant, particularly, wherein the sRNA is endogenously expressed in the plant.

8. The viral vector according to any one of claims 5-7 wherein:
- the plant is from the genus *Arabidopsis sp.,* particularly is *Arabidopsis thaliana,* and the sRNA is AtmiR173, more particularly, wherein the sequence of region R1 consists of SEQ ID NO. 2 (GUGAUUUUUCUCUACAAGCGAA),
- the plant of interest is from the genus *Nicotiana sp.,* particularly is *Nicotiana benthamiana* and the miRNA is NbmiR482a or NbmiR6019a, more particularly, wherein the sequence of region R1 is SEQ ID NO. 3 (GUGGUAUGGGGGGAGUCGGGAA) or SEQ ID NO. 4 (AAACAUUUACAAGUCACCUGUA), or
- the plant of interest is from the genus *Solanum,* particularly is *Solanum lycopersicum* and the miRNA is SlmiR482b or SlmiR6020, more particularly, wherein the sequence of region R1 is SEQ ID NO. 5 (GGCAUGGGCGGUGUAGGCAAGA) or SEQ ID NO. 6 (AAAGAUACUCGGAAAACAUUUA)

9. A virus encoded by or comprising the viral vector according to any one of claims 1-8.

10. An RNA polynucleotide comprising the RNA sequence S1 as defined in any one of claims 1-8 provided that the RNA polynucleotide does not comprise:
(i) in the region upstream of region R1, in a 5'- to 3'-end direction, the sequence immediately upstream the miR173 target site in the *Arabidopsis thaliana trans-acting siRNA1C* (*AtTAS1C*) primary precursor, and/or
(ii) in the region downstream of region R3, in a 5'- to 3'-end direction, the sequence immediately downstream sequence SEQ ID NO. 26 (gaacuagaaaagacauuggac) in the *AtTAS1C* primary precursor.

11. A plasmid comprising a sequence that encodes or corresponds to the sequence of the viral vector as defined in any one of claims 1-8., or that encodes the polynucleotide as defined in claim 9, particularly wherein the sequence that encodes or corresponds to the sequence of the viral vector, or the sequence that encodes the polynucleotide, is operatively linked to the sequence of an expression promoter.

12. A Bacterium that comprises the plasmid according to claim 11, particularly wherein the bacterium is from the species *Agrobacterium tumefaciens.*

13. A plant that comprises the viral vector according to any one of claims 1-8, the virus according to claim 9, the polynucleotide according to claim 10, the plasmid according to claim 11, and/or the bacterium according to claim 12, particularly wherein the plant is a non-transgenic plant.

14. A plant extract from a plant as defined in claim 13, particularly wherein the plant extract comprises the viral vector according to any one of claims 1-8 or the virus according to claim 9.

15. A method for obtaining the plant according to claim 13 that comprises the step of:
(i) transfecting a plant with the plasmid as defined in claim 11, particularly wherein transfection of the plasmid is performed by agroinfiltration with the bacterium as defined in claim 12, or alternatively,
(ii) contacting the surface of at least one organ of a plant with the virus as defined in claim 9, wherein contacting the surface of at least one organ of the plant with the virus comprises spraying the surface of said organ with the plant extract as defined in claim 14.
